# EUROPEAN PATENT APPLICATION

(11) **EP 4 445 914 A1**
(43) Date of publication of application: **16.10.2024**
(21) Application number: 22903570.4
(22) Date of filing: 08.12.2022
(51) Int. Cl.: A61K 45/06, A61K 31/506, A61K 31/704, A61K 39/395, A61P 35/02

(54) **PHARMACEUTICAL COMPOSITION FOR TREATING TUMORS AND USE**

(30) Priority: 10.12.2021 CN 202111508073; 22.09.2022 CN 202211160418; 02.12.2022 CN 202211543036
(71) Applicant: Inxmed (Nanjing) Co., Ltd., Nanjing, Jiangsu 210061 (CN)
(72) Inventor: ZHANG, Baoyuan, Nanjing, Jiangsu 210061 (CN); LIU, Xuebin, Nanjing, Jiangsu 210061 (CN); WANG, Zaiqi, Nanjing, Jiangsu 210061 (CN)
(74) Representative: D Young & Co LLP
(86) International application number: PCT/CN2022/137545
(87) International publication number: WO 2023/104151

(57) **Abstract**

The present invention relates to a combination of an FAK inhibitor, an anthracycline chemotherapeutic drug, and an immune checkpoint inhibitor to treat tumors. The present invention also relates to a combination of an FAK inhibitor and an anthracycline chemotherapeutic drug to treat tumors.

## Description

### TECHNICAL FIELD

The present disclosure belongs to the field of medicinal chemistry. Specifically, the present disclosure relates to the treatment of a tumor using a focal adhesion kinase (FAK) inhibitor in combination with other drugs.

### BACKGROUND

Tumor is the second largest killer threatening people's health. Immunogenic cell death (ICD) is based on the superposition effect of programmed cell death. An intracellular pressure signal may be activated when cancer cells are exposed to chemotherapy or targeted medicaments. This kind of pressure signal includes endoplasmic reticulum pressure (ER stress) and reactive oxygen species pressure (oxidative stress). Under the action of the pressure signal, cells will try to repair the pressure first. The cells will start the programmed death process, if the damage caused by the pressure exceeds the repair ability of the cells. This process is often accompanied by the release of a class of damage associated molecular patterns (DAMPs). This kind of DAMPs will be specifically recognized by pattern recognition receptors on antigen-presenting cells (APC) in the body, which will induce the maturation, differentiation and activation of APC, and then gradually present it to immune cells such as effector T cells, thus making immune cells generate antigen memory. When tumor cells from the same source are found again, immune cells will specifically recognize and kill the tumor cells. The new tumor-specific immune response initiated by ICD can increase the sensitivity to an immune checkpoint inhibitor (ICI), so as to enhance the effect of the immune checkpoint inhibitor, and produce anti-tumor response with lasting immune memory.

FAK, also known as protein tyrosine kinase 2 (PTK2), is a non-receptor tyrosine kinase and a key component of focal adhesion complex. FAK plays an important role in mediating integrin and growth factor signals to regulate the invasion, proliferation and survival of tumor cells.

Some chemotherapy drugs have been observed to trigger ICD of cancer cells, and ICD is understood to contribute to the anti-tumor effect of the therapy. Importantly, ICD stimulates the immune system to attack cancer, and may produce long-lasting anti-tumor effect particularly in combination with anti-PD1 or PD-L1. It has been reported in literature that PLD (doxorubicin hydrochloride liposome injection (doxil)) can induce ICD, but no significant clinical effect has been observed in clinical trials no matter it is combined with anti-PD-1 or PD-L1. Western Arizona Regional Medical Center explored a combination of anti-PD1 (pembrolizumab) and chemotherapy (including PLD) to treat tumors, but the clinical effect did not meet the expectations and the clinical trial has been terminated. Therefore, there is still a need to further search for drugs that can enhance the induction of ICD by chemotherapy drugs, and to improve the response rate of tumor treatment and the possibility of long-term benefit.

### SUMMARY

One aspect of the present disclosure provides use of an FAK inhibitor, an anthracycline chemotherapy drug, and an immune checkpoint inhibitor in the manufacture of a medicament for treating a tumor in a subject, wherein the FAK inhibitor, the anthracycline chemotherapy drug and the immune checkpoint inhibitor are administered to the subject simultaneously or sequentially.

Another aspect of the present disclosure provides a pharmaceutical combination product of an FAK inhibitor, an anthracycline chemotherapy drug, and an immune checkpoint inhibitor, for treating a tumor in a subject, wherein the FAK inhibitor, the anthracycline chemotherapy drug and the immune checkpoint inhibitor are administered to the subject simultaneously or sequentially.

One aspect of the present disclosure provides a method of treating a tumor, comprising administering a therapeutically effective amount of an FAK inhibitor, an anthracycline chemotherapy drug, and an immune checkpoint inhibitor simultaneously or sequentially to a subject in need.

Another aspect of the present disclosure provides a kit or a pharmaceutically acceptable composition, comprising: (a) an FAK inhibitor; (b) an anthracycline chemotherapy drug; and (c) an immune checkpoint inhibitor.

Another aspect of the present disclosure provides use of an FAK inhibitor and an anthracycline chemotherapy drug in the manufacture of a medicament for treating a tumor, wherein the FAK inhibitor is used to enhance the immunogenic cell death induced by the anthracycline chemotherapy drug.

Another aspect of the present disclosure provides an FAK inhibitor, for enhancing the immunogenic cell death induced by an anthracycline chemotherapy drug in the treatment of a tumor.

Another aspect of the present disclosure provides a method of treating a tumor, comprising administering a therapeutically effective amount of an FAK inhibitor and an anthracycline chemotherapy drug simultaneously or sequentially to a subject in need, wherein the FAK inhibitor is used to enhance the immunogenic cell death induced by the anthracycline chemotherapy drug.

Another aspect of the present disclosure provides use of an FAK inhibitor, an anthracycline chemotherapy drug, and an immune checkpoint inhibitor in the manufacture of a medicament for the combined treatment of a tumor.

Another aspect of the present disclosure provides use of an FAK inhibitor in the manufacture of a combined medicament with an anthracycline chemotherapy drug and an immune checkpoint inhibitor for treating a tumor.

Another aspect of the present disclosure provides use of an anthracycline chemotherapy drug in the manufacture of a combined medicament with an FAK inhibitor and an immune checkpoint inhibitor for treating a tumor.

Another aspect of the present disclosure provides use of an immune checkpoint inhibitor in the manufacture of a combined medicament with an FAK inhibitor and an anthracycline chemotherapy drug for treating a tumor.

Another aspect of the present disclosure provides use of an FAK inhibitor, an anthracycline chemotherapy drug, and an immune checkpoint inhibitor in the manufacture of a medicament for the combined treatment of a tumor.

Another aspect of the present disclosure provides use of an FAK inhibitor in the manufacture of a medicament for the combined treatment of a tumor with an anthracycline chemotherapy drug and an immune checkpoint inhibitor.

Another aspect of the present disclosure provides use of an anthracycline chemotherapy drug in the manufacture of a medicament for the combined treatment of a tumor with an FAK inhibitor and an immune checkpoint inhibitor.

Another aspect of the present disclosure provides use of an immune checkpoint inhibitor in the manufacture of a medicament for the combined treatment of a tumor with an FAK inhibitor and an anthracycline chemotherapy drug.

Another aspect of the present disclosure provides a kit comprising: an FAK inhibitor; and instructions, which indicate that the FAK inhibitor can be used for the combined treatment of a tumor with an anthracycline chemotherapy drug and an immune checkpoint inhibitor.

Another aspect of the present disclosure provides a kit comprising: an anthracycline chemotherapy drug; and instructions, which indicate that the anthracycline chemotherapy drug can be used for the combined treatment of a tumor with an FAK inhibitor and an immune checkpoint inhibitor.

Another aspect of the present disclosure provides a kit comprising: an immune checkpoint inhibitor; and instructions, which indicate that the immune checkpoint inhibitor can be used for the combined treatment of a tumor with an FAK inhibitor and an anthracycline chemotherapy drug.

Another aspect of the present disclosure provides a method of treating a tumor, comprising administering a therapeutically effective amount of an FAK inhibitor and an anthracycline chemotherapy drug to a subject in need.

Another aspect of the present disclosure provides a pharmaceutical combination product of an FAK inhibitor and an anthracycline chemotherapy drug, which is used for treating a tumor in a subject in need.

Another aspect of the present disclosure provides use of an FAK inhibitor and an anthracycline chemotherapy drug in the manufacture of a combined medicament for treating a tumor.

Another aspect of the present disclosure provides use of an FAK inhibitor in the manufacture of a combined medicament with an anthracycline chemotherapy drug for treating a tumor.

Another aspect of the present disclosure provides use of an anthracycline chemotherapy drug in the manufacture of a combined medicament with an FAK inhibitor for treating a tumor.

Another aspect of the present disclosure provides use of an FAK inhibitor and an anthracycline chemotherapy drug in the manufacture of a medicament for the combined treatment of a tumor.

Another aspect of the present disclosure provides use of an FAK inhibitor in the manufacture of a medicament for the combined treatment of a tumor with an anthracycline chemotherapy drug.

Another aspect of the present disclosure provides use of an anthracycline chemotherapy drug in the manufacture of a medicament for the combined treatment of a tumor with an FAK inhibitor.

Another aspect of the present disclosure provides a kit comprising: an FAK inhibitor; and instructions, which indicate that the FAK inhibitor can be used for the combined treatment of a tumor with an anthracycline chemotherapy drug.

Another aspect of the present disclosure provides a kit comprising: an anthracycline chemotherapy drug; and instructions, which indicate that the anthracycline chemotherapy drug can be used for the combined treatment of a tumor with an FAK inhibitor.

One aspect of the present disclosure provides use of an FAK inhibitor, Adriamycin, and an immune checkpoint inhibitor in the manufacture of a medicament for treating a tumor in a subject, wherein the FAK inhibitor, the Adriamycin, and the immune checkpoint inhibitor are administered to the subject simultaneously or sequentially.

Another aspect of the present disclosure provides a pharmaceutical combination product of an FAK inhibitor, Adriamycin and an immune checkpoint inhibitor for treating a tumor in a subject, wherein the FAK inhibitor, the Adriamycin and the immune checkpoint inhibitor are administered to the subject simultaneously or sequentially.

One aspect of the present disclosure provides a method of treating a tumor, comprising administering a therapeutically effective amount of an FAK inhibitor, Adriamycin, and an immune checkpoint inhibitor simultaneously or sequentially to a subject in need.

Another aspect of the present disclosure provides a kit or a pharmaceutically acceptable composition, comprising: (a) an FAK inhibitor; (b) Adriamycin; and (c) an immune checkpoint inhibitor.

Another aspect of the present disclosure provides use of an FAK inhibitor and Adriamycin in the manufacture of a medicament for treating a tumor, wherein the FAK inhibitor is used to enhance the immunogenic cell death induced by the Adriamycin.

Another aspect of the present disclosure provides an FAK inhibitor, for enhancing the immunogenic cell death induced by Adriamycin in the treatment of a tumor.

Another aspect of the present disclosure provides a method of treating a tumor, comprising administering a therapeutically effective amount of an FAK inhibitor and Adriamycin simultaneously or sequentially to a subject in need, wherein the FAK inhibitor is used to enhance the immunogenic cell death induced by the Adriamycin.

Another aspect of the present disclosure provides use of an FAK inhibitor, Adriamycin, and an immune checkpoint inhibitor in the manufacture of a medicament for the combined treatment of a tumor.

Another aspect of the present disclosure provides use of an FAK inhibitor in the manufacture of a combined medicament with Adriamycin and an immune checkpoint inhibitor for treating a tumor.

Another aspect of the present disclosure provides use of Adriamycin in the manufacture of a combined medicament with an FAK inhibitor and an immune checkpoint inhibitor for treating a tumor.

Another aspect of the present disclosure provides use of an immune checkpoint inhibitor in the manufacture of a combined medicament with an FAK inhibitor and Adriamycin for treating a tumor.

Another aspect of the present disclosure provides use of an FAK inhibitor, Adriamycin, and an immune checkpoint inhibitor in the manufacture of a medicament for the combined treatment of a tumor.

Another aspect of the present disclosure provides use of an FAK inhibitor in the manufacture of a medicament for the combined treatment of a tumor with Adriamycin and an immune checkpoint inhibitor.

Another aspect of the present disclosure provides use of Adriamycin in the manufacture of a medicament for the combined treatment of a tumor with an FAK inhibitor and an immune checkpoint inhibitor.

Another aspect of the present disclosure provides use of an immune checkpoint inhibitor in the manufacture of a medicament for the combined treatment of a tumor with an FAK inhibitor and Adriamycin.

Another aspect of the present disclosure provides a kit comprising: an FAK inhibitor; and instructions, which indicate that the FAK inhibitor can be used for the combined treatment of a tumor with Adriamycin and an immune checkpoint inhibitor.

Another aspect of the present disclosure provides a kit comprising: Adriamycin; and instructions, which indicate that Adriamycin can be used for the combined treatment of a tumor with an FAK inhibitor and an immune checkpoint inhibitor.

Another aspect of the present disclosure provides a kit comprising: an immune checkpoint inhibitor; and instructions, which indicate that the immune checkpoint inhibitor can be used for the combined treatment of a tumor with an FAK inhibitor and Adriamycin.

Another aspect of the present disclosure provides a method of treating a tumor, comprising administering a therapeutically effective amount of an FAK inhibitor and Adriamycin to a subject in need.

Another aspect of the present disclosure provides a pharmaceutical combination product of an FAK inhibitor and Adriamycin, which is used for treating a tumor in a subject in need.

Another aspect of the present disclosure provides use of an FAK inhibitor and Adriamycin in the manufacture of a combined medicament for treating a tumor.

Another aspect of the present disclosure provides use of an FAK inhibitor in the manufacture of a combined medicament with Adriamycin for treating a tumor.

Another aspect of the present disclosure provides use of Adriamycin in the manufacture of a combined medicament with an FAK inhibitor for treating a tumor.

Another aspect of the present disclosure provides use of an FAK inhibitor and Adriamycin in the manufacture of a medicament for the combined treatment of a tumor.

Another aspect of the present disclosure provides use of an FAK inhibitor in the manufacture of a medicament for the combined treatment of a tumor with Adriamycin.

Another aspect of the present disclosure provides use of Adriamycin in the manufacture of a medicament for the combined treatment of a tumor with an FAK inhibitor.

Another aspect of the present disclosure provides a kit comprising: an FAK inhibitor; and instructions, which indicate that the FAK inhibitor can be used for the combined treatment of a tumor with Adriamycin.

Another aspect of the present disclosure provides a kit comprising: Adriamycin; and instructions, which indicate that the Adriamycin can be used for the combined treatment of a tumor with an FAK inhibitor.

Optionally, the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 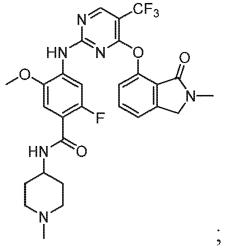 the Defactinib is also known as difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Ascentage Pharma; and the CAS number of AMP945 is 1393653-34-3.

Optionally, the anthracycline chemotherapy drug is Adriamycin, epirubicin, or daunorubicin, alternatively, the anthracycline chemotherapy drug is Adriamycin.

Optionally, the immune checkpoint inhibitor is an anti-PD-1/PD-L1 antibody, a PD-1/PD-L1 small molecule inhibitor, or a TIGIT inhibitor.

Optionally, the immune checkpoint inhibitor is an anti-PD-1/PD-L1 antibody. For example, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181, alternatively, the anti-PD-1/PD-L1 antibody is Toripalimab.

Optionally, the immune checkpoint inhibitor is a PD-1/PD-L1 small molecule inhibitor. For example, the PD-1/PD-L1 small molecule inhibitor is INCB-086550, Lazertinib, IMMH-010, CA-170, ABSK043, or RRx-001.

Optionally, the immune checkpoint inhibitor is a TIGIT inhibitor. For example, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

Optionally, the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer. For example, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; further, the tumor is ovarian cancer or colorectal cancer.

### BRIEF DESCRIPTION OF DRAWINGS

In order to more clearly explain the technical solution of examples of the present disclosure, the drawings of examples will be briefly introduced below. Obviously, the drawings in the following description only relate to some examples of the present disclosure, and do not limit the present disclosure.
FIG. 1 shows the knockdown effect of FAKsiRNA on FAK and phosphorylated FAKY397.
FIG. 2 shows the synergistic killing effect of the combined treatment of 3 µM Adriamycin and 50 nM FAKsiRNA for knockdown on ovarian cancer cells SK-OV-3.
FIG. 3 shows the immunofluorescence staining of DAMPs after 48 hours of combined treatment of 3 µM Adriamycin and 50 nM FAKsiRNA.
FIG. 4 shows 72-hour killing curves and IC₅₀ values of IN10018 and Adriamycin on ovarian cancer cells SK-OV-3.
FIG. 5 shows the synergistic killing effect of the combined treatment of Adriamycin at different dosages and 3 µM IN10018 for 48 hours on ovarian cancer cells SK-OV-3.
FIG. 6 shows the results of staining with Annexin V kit after 48 hours of combined treatment of 0.3 or 1.0 µM Adriamycin and 3 µM IN10018.
FIG. 7 shows the results of immunofluorescence staining of DAMPs after 48 hours of combined treatment of 0.3 µM Adriamycin and 3 µM IN10018.
FIG. 8 shows the results of immunofluorescence staining of DAMPs after 48 hours of combined treatment of 0.3 µM Daunorubicin or Epirubicin and 3 µM IN10018.
FIG. 9 shows the synergistic killing effect of the combined treatment of Adriamycin at different dosages and 3 µM Defactinib for 72 hours on ovarian cancer cells SK-OV-3.
FIG. 10 shows the results of immunofluorescence staining of DAMPs after 48 hours of combined treatment of 1 µM Adriamycin and 3 µM Defactinib.
FIG. 11 shows a bright view microscope photograph of cells incubated with a medicament for 48 hours.
FIG. 12 shows the percentages of CRT positive and Annexin V positive CT26 cells after 48 hours of incubation with IN10018 and Adriamycin, in which FIG. 12a shows the percentage of CRT positive CT26 cells after 48 hours of incubation with the medicament, and FIG. 12b shows the percentage of Annexin V positive CT26 cells after 48 hours of incubation with the medicament.
FIG. 13 shows the changes in tumor growth of the tumor-bearing mice after administration of different assay substances in the allograft tumor model of mouse colorectal cancer CT26 in BALB/c mice. A data point represents mean and standard error of the mean in the group (n = 8).
FIG. 14 shows the situation of changes in body weight of mice after administration of different dosages of a medicament and the changes in body weight of mice after administration of different dosages of a medicament. A data point represents mean ± standard error of the mean in the group (n = 8).
FIG. 15 shows the changes in tumor growth of the tumor-bearing mice after administration of different assay substances in the allograft tumor model of mouse colorectal cancer CT26 in BALB/c mice. A data point represents mean and standard error of the mean in the group (n = 8).
FIG. 16 shows the situation of changes in body weight of mice after administration of different dosages of a medicament and the changes in body weight of mice after administration of different dosages of a medicament. A data point represents mean ± standard error of the mean in the group (n = 8).
FIG. 17 shows the IFN-γ protein concentration detected in the serum isolated from a collected orbital blood 7 days after administration to the subcutaneous allograft tumor model of mouse colon cancer cell CT26 in BABL/c mice.
FIG. 18 shows the Granzyme B protein concentration detected in the serum isolated from a collected orbital blood 7 days after administration to the subcutaneous allograft tumor model of mouse colon cancer cell CT26 in BABL/c mice.
FIG. 19 shows the percentages of CRT positive and Annexin V positive 4T1 cells after 48 hours of incubation with Adriamycin and AMP945, in which FIG. 19a shows the percentage of CRT positive 4T1 cells after 48 hours of incubation with the medicament, and FIG. 19b shows the percentage of Annexin V positive 4T1 cells after 48 hours of incubation with the medicament.
FIG. 20 shows the percentages of CRT positive and Annexin V positive CT26 cells after 48 hours of incubation with Adriamycin and AMP945, in which FIG. 20a shows the percentage of CRT positive CT26 cells after 48 hours of incubation with the medicament, and FIG. 20b shows the percentage of Annexin V positive CT26 cells after 48 hours of incubation with the medicament.
FIG. 21 depicts a waterfall plot of the optimal change (%) in the total diameter of the target lesions relative to the baseline in the clinical trial of treating ovarian cancer using IN10018 combined with PLD and anti-PD-1.

### DETAILED DESCRIPTION

In order to make the purpose, technical solutions and advantages of the examples of the present disclosure clearer, the technical solutions of the examples of the present disclosure will be described clearly and completely with the attached figures of the examples of the present disclosure. Obviously, the described examples are parts of examples of the present disclosure, not the whole examples. Based on the described examples of the present disclosure, all other examples obtained by those of ordinary skill in the field without creative labor belong to the scope of protection of the present disclosure.

The present disclosure can be embodied in other specific forms without departing from the basic attributes of the present disclosure. It should be understood that any and all embodiments of the present disclosure can be combined with the technical features of any other embodiment or a plurality of other embodiments to obtain additional embodiments without conflict. The present disclosure includes the further embodiments resulting from such combinations.

All publications and patents mentioned in this disclosure are hereby incorporated by reference in their entirety. If the use or terminology used in any publication and patent incorporated by reference conflicts with the use or terminology used in this disclosure, the use and terminology of the present disclosure shall prevail.

The chapter headings used in this disclosure are only for the purpose of organizing articles and should not be interpreted as limitations on the subject.

Unless otherwise specified, all technical and scientific terms used herein have the usual meaning in the field to which the claimed subject matter belongs. If there are multiple definitions of a term, the definition in the present disclosure shall prevail.

Except in working examples or otherwise indicated, all numbers of quantitative properties such as dosage stated in the description and claims should be understood as being modified in all instances by the term "about". It should also be understood that any numerical range recited in this application is intended to include all subranges within the range and any combination of endpoints of the range or subrange.

"Including", "containing", "comprising" or the like used in this disclosure means that the elements appearing before the word cover the elements listed after the word and their equivalents, but do not exclude elements that are not recorded. The term "containing" or "including (comprising)" used herein can be open, semi-closed or closed. In other words, the term also includes "consisting essentially of" or "consisting of".

### Definition

The following terms and symbols used in this application have the following meanings, unless otherwise specified in the context. As used herein, the term "FAK inhibitor" refers to an effective inhibitor of FAK, which can be suitable for mammals, especially humans. In some embodiments, the FAK inhibitor is IN10018, Defactinib, GSK2256098, PF-00562271, VS-4718, APG-2449, AMP945, AMP886, or a pharmaceutically acceptable salt thereof, wherein the structure of IN10018 is as follows: 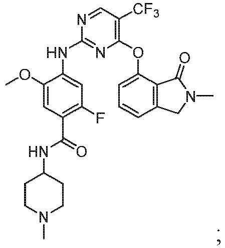 the Defactinib is also known as difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Ascentage Pharma; the CAS number of AMP945 is 1393653-34-3. In some embodiments, the FAK inhibitor is alternatively IN10018, Defactinib, AMP945, or a pharmaceutically acceptable salt thereof, and in some alternative embodiments, the FAK inhibitor is IN10018 or a pharmaceutically acceptable salt thereof, alternatively, the FAK inhibitor is IN10018 tartrate.

As used herein, the term "anthracycline drug" refers to a class of chemotherapy drugs derived from Streptomyces peucetius var. caesius. In some embodiments, the anthracycline drug is Adriamycin, epirubicin, daunorubicin, aclarithromycin, or the like. In some alternative embodiments, the anthracycline drug is Adriamycin.

The term "Adriamycin" used herein refers to doxorubicin. It can be understood that although the technical method of the present disclosure only mentions Adriamycin, in fact, doxorubicin or different salts or formulations thereof all fall within the scope of the claims of the present disclosure, for example, PLD, which refers to doxorubicin hydrochloride liposome injection (doxil), and is an Adriamycin hydrochloride liposome injection with an active ingredient of Adriamycin.

As used herein, the term "immune checkpoint inhibitor" refers to a medicament that can improve the immune system activity by regulating immune checkpoint pathways (such as PD-1, TIGIT, CTLA-4, LAG-3, TIM-3, etc.). In some embodiments, the immune checkpoint inhibitor is an antagonist of the PD-1/PD-L1 (programmed cell death protein 1) pathway (also called "PD-1 inhibitor"), or a TIGIT inhibitor. PD-1 inhibitors are also referred to as PD-1/PD-L1 inhibitors in this disclosure. For example, in the therapeutic methods, medicaments and uses of the present disclosure, the PD-1/PD-L1 inhibitor is an anti-PD-1/PD-L1 antibody, including, but not limited to, Pembrolizumab (kerida/Keytruda/K medicament), Tislelizumab (Baizean), Nivolumab, Toripalimab (Tuoyi), Durvalumab (Imfinzi), Avelumab (Bavencio), Atezolizumab (MPDL 3280A/Tecentriq/T medicament), BMS-936559 (fully humanized IgG4 monoclonal antibody against PD-L1), GS-4224, AN-4005, or MX-10181, particularly Toripalimab. In some alternative embodiments, the PD-1 inhibitor is Toripalimab. In some embodiments, the PD-1 inhibitor is used to treat a human subject. In some embodiments, the PD-1 is human PD-1. The PD-1/PD-L1 inhibitors also include PD-1/PD-L1 small molecule inhibitors, such as INCB-086550, Lazertinib, IMMH-010, CA-170, ABSK043, or RRx-001. TIGIT (also known as WUCAM, Vstm3, or VSIG9) is a receptor of the Ig superfamily and is a new immune checkpoint besides PD-1/PD-L1. For example, in the therapeutic methods, medicaments and uses of the present disclosure, the immune checkpoint inhibitor is a TIGIT inhibitor (e.g., TIGIT antibody), including, but not limited to, Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321. In some embodiments, the TIGIT inhibitor is used to treat a human subject. In order to avoid ambiguity, all antibodies in the present disclosure include bispecific antibodies.

As used herein, "pharmaceutical combination" or "pharmaceutical combination product" can refer to the case of a fixed combination in the form of a dosage unit (for example, all pharmaceutical active ingredients exist in one dosage form) or the case of a product that is administered in combination in a complete kit, and the case of a combination of a medicament and instructions indicating that the medicament can be used in combination with one or more other medicaments.

As used herein, "combination therapy" or "combined medicament" means that a medicament is used in combination with one or more other medicaments to treat a disease, including both the combination of one medicament and one or more other medicaments and the combination of one medicament and instructions indicating that the medicament can be used in combination with one or more other medicaments.

In this application, "simultaneous or sequential administration" refers to the simultaneous administration of two or more medicaments or sequential administration of two or more medicaments at a certain time interval within an administration cycle (for example, within 4 weeks, 3 weeks, 2 weeks, 1 week, or 24 hours). The modes of administration (for example, oral administration, intravenous administration, intramuscular administration, or subcutaneous administration) may be the same or different, and the frequency/cycle of administration of two or more medicaments may be the same or different. When the therapeutic method, product or use of the present disclosure involves two medicaments, the two medicaments can be administered simultaneously or separately at a certain interval. When the therapeutic method, product or use of the present disclosure involves three medicaments, the three medicaments can be administered at the same time point, or two medicaments can be administered at one time point and the remaining one medicament can be administered at another time point, or each of the three medicaments can be administered at different time points.

In some embodiments, the PD-1/PD-L1 inhibitor is administered intravenously (e.g., as intravenous infusion), subcutaneously, or orally. Alternatively, the PD-1/PD-L1 inhibitor is administered by intravenous infusion.

The ability of immune checkpoint inhibitors to treat cancer depends on the existence of tumor antigen-specific T cells in a tumor tissue. This requires tumor tissues to express antigens that distinguish themselves from their untransformed counterparts, for example, through a new protein product called neoantigen. The neoantigen load of tumor is closely related to immunogenicity and sensitivity (for example, sensitivity to checkpoint inhibitor therapy), which means that tumors with poor immunogenicity should be resistant to these medicaments to a great extent. Therapeutics for releasing tumor antigens that can be ingested by APC, such as those that induce immunogenic cell death (ICD), may promote effective anti-tumor immunity, especially when further combined with a checkpoint inhibitor.

As used herein, the term "treatment" refers to the administration of one or more pharmaceutical substances to a subject suffering from a disease or having symptoms of the disease in order to cure, alleviate, mitigate, change, treat, improve, ameliorate, or influence the disease or symptoms of the disease. In some embodiments, the disease is a tumor or cancer.

As used herein, the term "tumor" refers to the abnormal pathological changes formed by the abnormal proliferation of clonal cells caused by the loss of normal regulation of the growth of cells of local tissues at the gene level under the action of various tumorigenic factors. Examples include, but are not limited to: Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.

As used herein, the term "subject" or "patient" refers to mammals and non-mammals. Mammals refer to any member of mammals, including but not limited to: human; non-human primates, such as chimpanzees and other apes and monkey species; farm animals such as cattle, horses, sheep, goats and pigs; domestic animals such as rabbits, dogs and cats; laboratory animals, including rodents, such as rats, mice and guinea pigs, etc. Examples of non-mammals include but are not limited to birds, etc. The term "subject" is not limited to a specific age or gender. In some embodiments, the subject is a human.

As used herein, the term "pharmaceutically acceptable" means non-toxic, biologically tolerable and suitable for administration to a subject.

As used herein, the term "pharmaceutically acceptable salt" refers to non-toxic and biologically tolerable acid addition salts suitable for administration to subjects, including but not limited to: acid addition salts formed with inorganic acids, such as hydrochloride, hydrobromide, carbonate, bicarbonate, phosphate, sulfate, sulfite, nitrate, etc.; and acid addition salts formed with organic acids, such as formate, acetate, malate, maleate, fumarate, tartrate, succinate, citrate, lactate, methanesulfonate, p-toluenesulfonate, 2-hydroxyethyl sulfonate, benzoate, salicylate, stearate and salts formed with alkanedicarboxylic acids of formula HOOC-(CH₂)ₙ-COOH (wherein n is 0-4), etc.

Furthermore, a pharmaceutically acceptable acid addition salt can be obtained by dissolving a free base in a suitable solvent and treating the solution with an acid according to a conventional operation for preparing an acid addition salt from a basic compound. Those skilled in the art can determine various synthetic methods that can be used to prepare nontoxic pharmaceutically acceptable acid addition salts without redundant experiments.

As used herein, the term "pharmaceutically acceptable composition" means that it must be chemically and/or toxicologically compatible with other ingredients included in the preparation, and/or compatible with the subject to be treated. As used herein, the term "therapeutically effective amount" refers to an amount generally sufficient to produce a beneficial therapeutic effect on a subject. The therapeutically effective amount of the present disclosure can be determined by conventional methods (such as modeling, dose escalation study or clinical trial) combined with conventional influencing factors (such as administration mode, pharmacokinetics of compounds, severity and course of diseases, medical history of subjects, health status of subjects, response degree of subjects to medicaments, etc.).

As used herein, the term "inhibition" refers to a decrease in the baseline activity of a biological activity or process.

As used herein, the term "kit" refers to a box for containing chemical reagents for detecting chemical components, medicament residues, virus types and the like. The kit of the present disclosure can include (i) one, two or three of an FAK inhibitor, an anthracycline chemotherapy drug (e.g., Adriamycin) and an immune checkpoint inhibitor; and (ii) instructions indicating that the FAK inhibitor, the anthracycline chemotherapy drug and the immune checkpoint inhibitor can be used to treat a tumor in a subject. In one embodiment, the kit comprises (i) an FAK inhibitor; and (ii) instructions indicating that the FAK inhibitor, an anthracycline chemotherapy drug and an immune checkpoint inhibitor can be used to treat a tumor in a subject. In one embodiment, the kit comprises (i) an anthracycline chemotherapy drug; and (ii) instructions indicating that an FAK inhibitor, the anthracycline chemotherapy drug and an immune checkpoint inhibitor can be used to treat a tumor in a subject. In one embodiment, the kit comprises (i) an immune checkpoint inhibitor; and (ii) instructions indicating that an FAK inhibitor, an anthracycline chemotherapy drug and the immune checkpoint inhibitor can be used to treat a tumor in a subject. In one embodiment, the kit comprises (i) an FAK inhibitor, an anthracycline chemotherapy drug and an immune checkpoint inhibitor; and (ii) instructions indicating that the FAK inhibitor, the anthracycline chemotherapy drug and the immune checkpoint inhibitor can be used to treat a tumor in a subject. In one embodiment, the kit comprises (i) an FAK inhibitor; and (ii) instructions indicating that the FAK inhibitor and an anthracycline chemotherapy drug can be used to treat a tumor in a subject. In one embodiment, the kit comprises (i) an anthracycline chemotherapy drug; and (ii) instructions indicating that the anthracycline chemotherapy drug and an FAK inhibitor can be used to treat a tumor in a subject.

The compounds of a kit can be contained in separate containers. Optionally, two or more compounds are contained in the same container. For example, the kit may include a first container, a second container, a third container, and a package insert, wherein the first container includes at least one dose of a medicament including an FAK inhibitor, the second container includes at least one dose of an anthracycline chemotherapy drug (e.g. Adriamycin), and the third container includes at least one dose of a medicament including an immune checkpoint inhibitor, and the package insert includes instructions of treating a tumor in a subject with the medicament. The first container, the second container and the third container may contain the same or different shapes (e.g., vials, syringes and bottles) and/or materials (e.g., plastic or glass). The kit can also include other materials that can help to administer medicaments, such as diluents, filters, IV bags and lines, needles and syringes.

The exact amount of the FAK inhibitor, the anthracycline drug and the immune checkpoint inhibitor to be administered to a subject will depend on various factors, such as a given medicament or compound, pharmaceutical preparation, route of administration, type of disease, symptoms, identity of the subject or host to be treated, etc., but it can still be routinely determined by those skilled in the art. For example, determining the effective amount also depends on the degree, severity and type of cell proliferation. The skilled person will be able to determine the appropriate dosage based on these and other factors.

The FAK inhibitor, the anthracycline drug, and the immune checkpoint inhibitor can be administered in an appropriate manner, such as oral administration, intravenous administration, intramuscular administration, or subcutaneous administration.

For example, when administered orally, the medicament can be administered orally together with a pharmaceutically acceptable carrier such as an inert diluent or an absorbable edible carrier. They can be encapsulated in hard-shell or soft-shell gelatin capsules, can be pressed into tablets, or can be directly mixed with patients' food. For example, medicaments can be combined with one or more excipients and used in the form of ingestible tablets, oral tablets, lozenges, capsules, elixirs, suspensions, syrups, or wafers. Tablets, lozenges, pills, capsules, and the like may further include a binder, such as tragacanth gum, arabic gum, corn starch or gelatin; an excipient, such as dicalcium phosphate; a disintegrator, such as corn starch, potato starch, alginic acid, and the like; a lubricant, such as magnesium stearate; or a sweetener, such as sucrose, fructose, lactose or aspartame; or a flavoring agent.

For example, when administered intravenously or intraperitoneally by infusion or injection, the medicament solution can be prepared in water, optionally mixed with a non-toxic surfactant.

Exemplary pharmaceutical dosage forms for injection or infusion include a sterile aqueous solution, a dispersion, or a sterile powder containing an active ingredient, which is suitable for temporarily preparing a sterile injection or infusion solution or dispersion. In any case, the final dosage form should be sterile, fluid and stable under the conditions of production and storage.

Sterile injection solution can be prepared by mixing the required amount of medicaments with the required various other components mentioned above into a suitable solvent, and then filtering and sterilizing the mixture. For the sterile powder used to prepare the sterile injection solution, the alternative preparation methods can be vacuum drying and freeze-drying techniques, which can produce powder of active ingredient plus any other required ingredients existing after previous sterile filtration.

The amount of the FAK inhibitor, the anthracycline drug and the immune checkpoint inhibitor required for treatment can vary not only with the specific reagents selected, but also with the route of administration, the nature of the diseases to be treated, and the age and condition of the patients, and can be finally decided by the attending physician or clinician. However, in general, the dosage can be in the range of about 0.1 to about 50mg/kg body weight per day.

The FAK inhibitor is administered in a dosage range of 5mg/day to 300 mg/day in adults. In a specific embodiment, IN10018 or a pharmaceutically acceptable salt thereof is administered at a dosage of 5 mg/day to 100 mg/day in adults, and the dosage is calculated based on the free base.

The anthracycline drug is administered in a dosage range of 1 to 40 mg/m² per week in adults. In a specific embodiment, Adriamycin or a pharmaceutically acceptable salt thereof is administered at a dosage of 1 to 40 mg/m² per week in adults, and the dosage is calculated based on Adriamycin.

The immune checkpoint inhibitor is administered each time at a dosage of 2 to 10mg/kg or 50 to 1200mg in adults, and administered every 2 to 3 weeks. In a specific embodiment, the immune checkpoint inhibitor is administered each time at a dosage of 3 to 10mg/kg or 100 to 1200mg in adults, and administered every 2 to 3 weeks.

Technical and scientific terms not specifically defined used herein have the meanings commonly understood by those skilled in the art to which this disclosure belongs.

In some embodiments, the present disclosure also discloses:
1. An FAK inhibitor, an anthracycline drug and an immune checkpoint inhibitor, for use in a method of treating a tumor in a subject, wherein the FAK inhibitor, the anthracycline drug, and the immune checkpoint inhibitor are administered to the subject simultaneously or sequentially.
2. The compounds for use according to embodiment 1, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 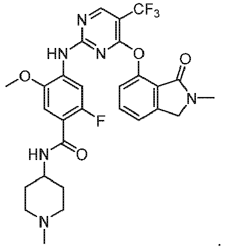
3. The compounds for use according to embodiment 1 or 2, wherein the anthracycline drug is Adriamycin, epirubicin, or daunorubicin, alternatively, the anthracycline drug is Adriamycin.
4. The compounds for use according to any one of embodiments 1 to 3, wherein the immune checkpoint inhibitor is an anti-PD-1/PD-L1 antibody, a PD-1/PD-L1 small molecule inhibitor, or a TIGIT inhibitor.
5. The compounds for use according to any one of embodiments 1 to 4, wherein the FAK inhibitor is IN10018 or a pharmaceutically acceptable salt thereof, the anthracycline drug is Adriamycin, and the immune checkpoint inhibitor is an anti-PD-1/PD-L1 antibody or a PD-1/PD-L1 small molecule inhibitor, alternatively, the immune checkpoint inhibitor is toripalimab.
6. The compounds for use according to any one of embodiments 1 to 5, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
7. A kit or a pharmaceutically acceptable composition, comprising:
   (a) an FAK inhibitor;
   (b) an anthracycline drug; and
   (c) an immune checkpoint inhibitor.
8. The kit or composition according to embodiment 7, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 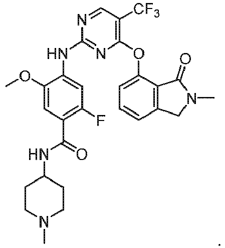
9. The kit or composition according to embodiment 7 or 8, wherein the anthracycline drug is Adriamycin, epirubicin, or daunorubicin, alternatively, the anthracycline drug is Adriamycin.
10. The kit or composition according to any one of embodiments 7 to 9, wherein the immune checkpoint inhibitor is an anti-PD-1/PD-L1 antibody, a PD-1/PD-L1 small molecule inhibitor, or a TIGIT inhibitor.
11. The kit or composition according to any one of embodiments 7 to 10, wherein the FAK inhibitor is IN10018 or a pharmaceutically acceptable salt thereof, the anthracycline drug is Adriamycin, and the immune checkpoint inhibitor is a PD-1/PD-L1 inhibitor, alternatively, the immune checkpoint inhibitor is toripalimab.
12. The kit or composition according to any one of embodiments 7 to 11, for use as a medicament.
13. The kit or composition according to any one of embodiments 7 to 12, for use in treating a tumor.
14. The kit or composition according to embodiment 13, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
15. A method of treating a tumor in a subject, wherein the method comprises administering the compounds of the kit or composition according to any one of embodiments 7 to 12 to the subject simultaneously or sequentially.
16. The method according to embodiment 15, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
17. An FAK inhibitor, for use in enhancing the immunogenic cell death induced by an anthracycline drug.
18. The FAK inhibitor for use according to embodiment 17, wherein the anthracycline drug is Adriamycin, epirubicin, or daunorubicin, alternatively, the anthracycline drug is Adriamycin.
19. The FAK inhibitor for use according to embodiment 17 or 18, which is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 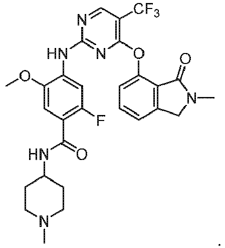
20. The FAK inhibitor for use according to any one of embodiments 17 to 19, wherein the FAK inhibitor and the anthracycline drug are administered simultaneously or sequentially.
21. A method of treating a tumor in a subject, wherein the method comprises administering a therapeutically effective amount of an FAK inhibitor, an anthracycline drug, and an immune checkpoint inhibitor simultaneously or sequentially to the subject.
22. The method according to embodiment 21, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 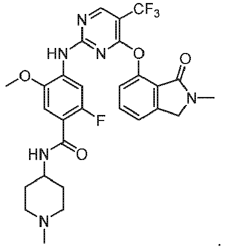
23. The method according to embodiment 21 or 22, wherein the anthracycline drug is Adriamycin, epirubicin, or daunorubicin, alternatively, the anthracycline drug is Adriamycin.
24. The method according to any one of embodiments 21 to 23, wherein the immune checkpoint inhibitor is a PD-1/PD-L1 inhibitor.
25. The method according to any one of embodiments 21 to 24, wherein the FAK inhibitor is IN10018 or a pharmaceutically acceptable salt thereof, the anthracycline drug is Adriamycin, and the immune checkpoint inhibitor is a PD-1/PD-L1 inhibitor, alternatively, the immune checkpoint inhibitor is toripalimab.
26. The method according to any one of embodiments 21 to 25, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
27. An FAK inhibitor, an anthracycline drug and an immune checkpoint inhibitor, for use in a method of treating a tumor by increasing immunogenic cell death in a subject, wherein the FAK inhibitor, the anthracycline drug and the immune checkpoint inhibitor are administered to the subject simultaneously or sequentially.
28. The compounds for use according to embodiment 27, wherein the FAK inhibitor is IN10018, defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 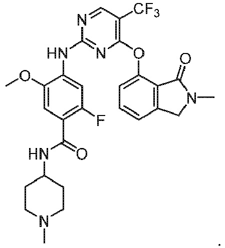
29. The method according to embodiment 27 or 28, wherein the anthracycline drug is Adriamycin, epirubicin, or daunorubicin, alternatively, the anthracycline drug is Adriamycin.
30. The compounds for use according to any one of embodiments 27 to 29, wherein the immune checkpoint inhibitor is an anti-PD-1/PD-L1 antibody, a PD-1/PD-L1 small molecule inhibitor, or a TIGIT inhibitor.
31. The compounds for use according to any one of embodiments 27 to 30, wherein the FAK inhibitor is IN10018 or a pharmaceutically acceptable salt thereof, the anthracycline drug is Adriamycin, and the immune checkpoint inhibitor is an anti-PD-1/PD-L1 antibody or a PD-1/PD-L1 small molecule inhibitor.
32. The compounds for use according to any one of embodiments 27 to 31, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
33. A method of treating a tumor by increasing immunogenic cell death in a subject, wherein the method comprises administering a therapeutically effective amount of an FAK inhibitor, an anthracycline drug, and an immune checkpoint inhibitor simultaneously or sequentially to the subject.
34. The method according to embodiment 33, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 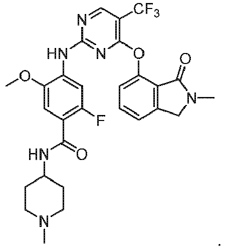
35. The method according to embodiment 33 or 34, wherein the anthracycline drug is Adriamycin, epirubicin, or daunorubicin, alternatively, the anthracycline drug is Adriamycin.
36. The method according to any one of embodiments 33 to 35, wherein the immune checkpoint inhibitor is an anti-PD-1/PD-L1 antibody, a PD-1/PD-L1 small molecule inhibitor, or a TIGIT inhibitor.
37. The method according to any one of embodiments 33 to 36, wherein the FAK inhibitor is IN10018 or a pharmaceutically acceptable salt thereof, the anthracycline drug is Adriamycin, and the immune checkpoint inhibitor is an anti-PD-1/PD-L1 antibody or a PD-1/PD-L1 small molecule inhibitor, alternatively, the immune checkpoint inhibitor is toripalimab.
38. The method according to any one of embodiments 33 to 37, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
39. Use of an FAK inhibitor in the manufacture of a medicament for treating a tumor in a subject, wherein the FAK inhibitor, an anthracycline drug, and an immune checkpoint inhibitor are administered to the subject simultaneously or sequentially.
40. The use according to embodiment 39, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 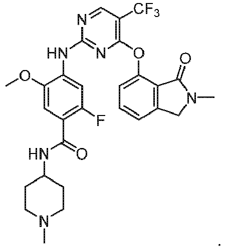
41. The use according to embodiment 39 or 40, wherein the anthracycline drug is Adriamycin, epirubicin, or daunorubicin, alternatively, the anthracycline drug is Adriamycin.
42. The use according to any one of embodiments 39 to 41, wherein the immune checkpoint inhibitor is a PD-1/PD-L1 inhibitor.
43. The use according to any one of embodiments 39 to 42, wherein the FAK inhibitor is IN10018 or a pharmaceutically acceptable salt thereof, the anthracycline drug is Adriamycin, and the immune checkpoint inhibitor is a PD-1/PD-L1 inhibitor, alternatively, the immune checkpoint inhibitor is toripalimab.
44. The use according to any one of embodiments 39 to 43, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
45. Use of an anthracycline drug in the manufacture of a medicament for treating a tumor in a subject, wherein an FAK inhibitor, the anthracycline drug and an immune checkpoint inhibitor are administered to the subject simultaneously or sequentially.
46. The use according to embodiment 45, wherein the anthracycline drug is Adriamycin, epirubicin, or daunorubicin, alternatively, the anthracycline drug is Adriamycin.
47. The use according to embodiment 45 or 46, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 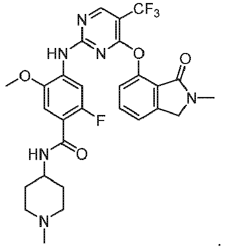
48. The use according to any one of embodiments 45 to 47, wherein the immune checkpoint inhibitor is an anti-PD-1/PD-L1 antibody, a PD-1/PD-L1 small molecule inhibitor, or a TIGIT inhibitor.
49. The use according to any one of embodiments 45 to 48, wherein the FAK inhibitor is IN10018 or a pharmaceutically acceptable salt thereof, the anthracycline drug is Adriamycin, and the immune checkpoint inhibitor is an anti-PD-1/PD-L1 antibody or a PD-1/PD-L1 small molecule inhibitor, alternatively, the immune checkpoint inhibitor is toripalimab.
50. The use according to any one of embodiments 45 to 49, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.
51. Use of an immune checkpoint inhibitor in the manufacture of a medicament for treating a tumor in a subject, wherein an FAK inhibitor, an anthracycline drug, and the immune checkpoint inhibitor are administered to the subject simultaneously or sequentially.
52. Use of an FAK inhibitor, an anthracycline chemotherapy drug and an immune checkpoint inhibitor in the manufacture of a combined medicament for treating a tumor.
53. Use of an FAK inhibitor in the manufacture of a combined medicament with an anthracycline chemotherapy drug and an immune checkpoint inhibitor for treating a tumor.
54. Use of an anthracycline chemotherapy drug in the manufacture of a combined medicament with an FAK inhibitor and an immune checkpoint inhibitor for treating a tumor.
55. Use of an immune checkpoint inhibitor in the manufacture of a combined medicament with an FAK inhibitor and an anthracycline chemotherapy drug for treating a tumor.
56. Use of an FAK inhibitor, an anthracycline chemotherapy drug, and an immune checkpoint inhibitor in the manufacture of a medicament for the combined treatment of a tumor.
57. Use of an FAK inhibitor in the manufacture of a medicament for the combined treatment of a tumor with an anthracycline chemotherapy drug and an immune checkpoint inhibitor.
58. Use of an anthracycline chemotherapy drug in the manufacture of a medicament for the combined treatment of a tumor with an FAK inhibitor and an immune checkpoint inhibitor.
59. Use of an immune checkpoint inhibitor in the manufacture of a medicament for the combined treatment of a tumor with an FAK inhibitor and an anthracycline chemotherapy drug.
60. The use according to any one of embodiments 51 to 59, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, and the structure of IN10018 is as follows: 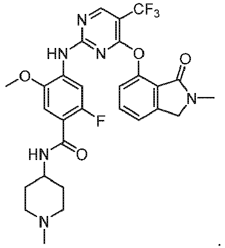
61. The use according to any one of embodiments 51 to 60, wherein the anthracycline drug is Adriamycin, epirubicin, or daunorubicin, alternatively, the anthracycline drug is Adriamycin.
62. The use according to any one of embodiments 51 to 61, wherein the immune checkpoint inhibitor is an anti-PD-1/PD-L1 antibody, a PD-1/PD-L1 small molecule inhibitor, or a TIGIT inhibitor.
63. The use according to any one of embodiments 51 to 62, wherein the FAK inhibitor is IN10018 or a pharmaceutically acceptable salt thereof, the anthracycline drug is Adriamycin, and the immune checkpoint inhibitor is an anti-PD-1/PD-L1 antibody or a PD-1/PD-L1 small molecule inhibitor, alternatively, the immune checkpoint inhibitor is toripalimab.
64. The use according to any one of embodiments 51 to 63, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or cholangiocarcinoma; yet further, the tumor is ovarian cancer or colorectal cancer.

### EXAMPLES

The following examples are provided to further illustrate the present disclosure. It should be understood that these examples are only used to illustrate the present disclosure and are not used to limit the scope of the present disclosure.

The assay methods without specific conditions in the following examples can be carried out according to the conventional conditions of this kind of reaction or according to the conditions suggested by the manufacturer.

The assay materials and reagents used in the following examples are all commercially available unless otherwise specified.

The abbreviations used in the examples have the following meanings:

| Abbreviation | Name |
|---|---|
| siRNA | Small interfering RNA |
| DMSO | Dimethyl sulfoxide |
| COMBO | Combination |
| HMGB1 | High mobility group box-1 protein |
| AAALAC | Association for Assessment and Accreditation of Laboratory Animal Care |
| IACUC | Institutional Animal Care and Use Committee |
| SPF | Specific pathogen free |
| BIW | Twice a week |
| BID | Twice a day |
| QD | Once a day |
| QW | Once a week |
| BW | Body weight |
| EDTA | Ethylenediaminetetraacetic acid |
| GLP | Good Laboratory Practice for Nonclinical Laboratory Studies |
| *p*.*o*. | Oral gavage |
| *i.v.* | Intravenous administration |
| *i.p.* | Intraperitoneal injection administration |
| DPBS | Dulbecco's Phosphate Buffered Saline |
| DDH₂O | Double distilled water |
| RT | Room temperature |

| | |
|---|---|
| SEM | Standard error of the mean |
| TGI | Tumor growth inhibition rate |
| TV | Tumor volume |
| TW | Tumor weight |
| FBS | Fetal bovine serum |
| PBS | Phosphate-buffered saline |
| MC | Methylcellulose |
| CO₂ | Carbon dioxide |
| CRT | Calreticulin |
| Annexin-V-FITC | Fluorescein isothiocyanate-labeled annexin |
| PI | Propidium iodide |

### Example 1: Study on the enhancement of immunogenic cell death of ovarian cancer cells in response to Adriamycin by FAK knockdown

### Assay protocol:

### 1) FAKsiRNA was used to knock down FAK and the knockdown efficiency was detected

SK-OV-3 cells were used as the research object, and Lipofectamine 3000 was used to transfect the cells with 50 nM FAKsiRNA. After the assay, proteins were collected to detect the expression of FAK and phosphorylated FAKY397.

### 2) FAK knockdown combined with Adriamycin produced a synergistic tumor killing effect

An in vitro assay was performed to detect the cell killing effect of the knockdown effect of 50 nM FAKsiRNA and 3 µM Adriamycin on ovarian cancer cells SK-OV-3. The cell death was photographed after 48 hours.

### 3) FAK knockdown combined with Adriamycin can effectively induce the ICD effect of ovarian cancer cells SK-OV-3

An in vitro assay was performed to test whether 48 hours of combined use of FAK knockdown and Adriamycin can effectively increase the release of Calreticulin and Annexin A1, the main targets of ICD, from cells.

### Assay materials:

### Drugs used in this assay:

FAKsiRNA was provided by GenePharma, with a sequence shown in the table below. Adriamycin was provided by MCE.

FAKsiRNA:

GGGCAUCAUUCAGAAGAUATT
UAUCUUCUGAAUGAUGCCCTT

### Antibodies used in this assay:

Recombinant Alexa Fluor^{®} 488 Anti-HMGB1 antibody (Abcam, ab195010); Recombinant Alexa Fluor^{®} 647 Anti-Calreticulin antibody (Abcam, ab196159); Annexin A1 rabbit antibody (Cell signaling technology, 32934S); Anti-rabbit IgG (H+L), F(ab')2 Fragment (Alexa Fluor^{®} 488 Conjugate) (Cell signaling technology, 4412); FAK antibody (CST, 3285S); Phospho-FAKY397 antibody (Thermo fisher, 44-624G); HRP-conjugated alpha tubulin antibody (Proteintech, HRP-66031).

### Assay instrument:

Fluorescence microscope (Olympus U-HGLGPS); Chemiluminescence imaging system (BIORAD chemidoc touch).

### Assay results:

### 1) FAKsiRNA was used to knock down FAK and the knockdown efficiency was detected

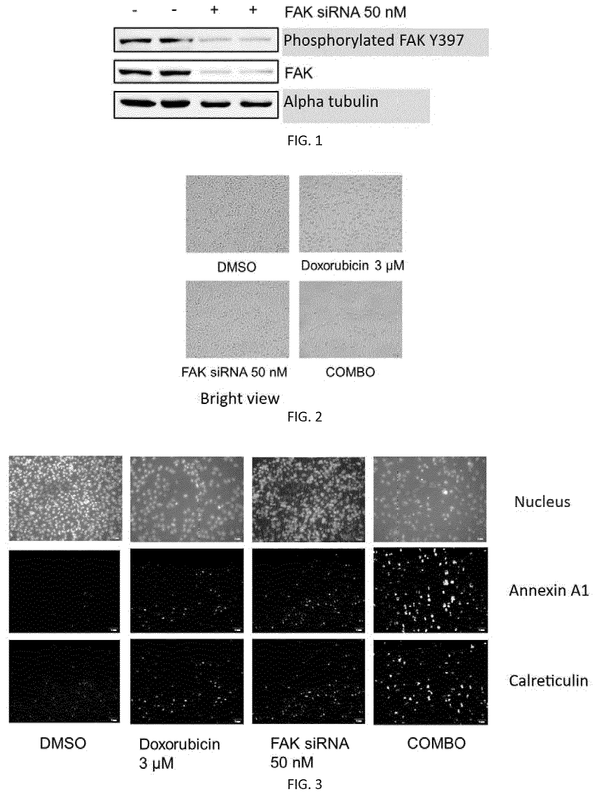

SK-OV-3 cells were transfected with 50 nM FAKsiRNA. Lipofectamine 3000 was used as a transfection reagent. Downstream combination and ICD biomarker assays can be performed 24 hours after the transfection. 72 hours after the transfection, SK-OV-3 cell proteins were collected, and western blot was performed to detect the amount of phosphorylated FAKY397, FAK and alpha tubulin. The effect of transfection was based on the protein knockdown as a standard, as shown in FIG. 1. FAKsiRNA had a significant knockdown effect on phosphorylated FAKY397 and total FAK proteins.

### 2) FAK knockdown combined with Adriamycin produced a synergistic tumor killing effect

SK-OV-3 cells were transfected with 50 nM FAKsiRNA for 24 hours and then 3 µM Adriamycin was added. After 48 hours of co-culture, the cells were photographed, and it was found that FAK knockdown combined with Adriamycin produced a significant synergistic killing effect on tumor cells. The results are shown in FIG. 2.

### 3) The combination of PLD and FAK knockdown significantly enhanced the release and exposure of Calreticulin and Annexin A1, the ICD targets of SK-OV-3

SK-OV-3 cells were treated with 3 µM Adriamycin combined with 50 nM FAKsiRNA. After 48 hours of treatment, antibodies against Hoechst 33342, Calreticulin, and Annexin A1 were used for fluorescent staining. The results are shown in FIG. 3, showing that compared with the single drug group, the release and exposure of Calreticulin and Annexin A1 were significantly enhanced in the combination group.

### Assay conclusion:

FAK knockdown can significantly enhance the cell killing effect of Adriamycin on ovarian cancer cells SK-OV-3. In the assay of detecting ICD markers, it was found that FAK knockdown can significantly enhance the exposure and release of DAMP markers Calreticulin and Annexin A1, suggesting that FAK knockdown can effectively enhance the immunogenic cell death in ovarian cancer cells.

### Example 2: Study on the enhancement of immunogenic cell death of ovarian cancer cells in response to Adriamycin by IN10018

### Assay protocol:

### 1) IN10018 combined with Adriamycin produced a synergistic tumor killing effect

An in vitro assay was conducted to detect the cell killing curve of IN10018 and Adriamycin in ovarian cancer cells SK-OV-3, and IC₅₀ values were determined. The cytotoxic effects of Adriamycin at different dosages combined with 3 µM IN10018 were explored. The Annexin V method was used to detect the apoptotic response to the medicaments used alone and in combination. Student's t-test was used in the statistical research, and **** means the P value is less than 0.001.

### 2) IN10018 combined with Adriamycin can effectively induce the ICD effect of ovarian cancer cells SK-OV-3

An in vitro assay was conducted to test whether 48 hours of combined use of IN10018 and Adriamycin can effectively increase the release of Calreticulin, HMGB1 and Annexin A1, the main targets of ICD, from cells.

### Assay materials:

### 1) Drugs used in this assay

IN10018 was synthesized according to the method in patent WO2010058032. Adriamycin was provided by MCE.

### 2) Antibodies used in this assay

Recombinant Alexa Fluor^{®} 488 Anti-HMGB1 antibody (Abcam, ab195010); Recombinant Alexa Fluor^{®} 647 Anti-Calreticulin antibody (Abcam, ab196159); Annexin A1 rabbit antibody (Cell signaling technology, 32934S); Anti-rabbit IgG (H+L), F(ab')2 Fragment (Alexa Fluor^{®} 488 Conjugate) (Cell signaling technology, 4412).

### 3) Kits used in this assay

Annexin V apoptosis detection kit (Abbkine, KTA0002); CCK8 cell viability reagent (Cellor lab, CX001).

### Assay instrument:

Microplate reader (Molecular devices M3); Fluorescence microscope (Olympus U-HGLGPS); Flow cytometer (BD LSRFortessa X-20).

### Assay results:

### 1) IC₅₀ curves of IN10018 and Adriamycin used alone in SK-OV-3

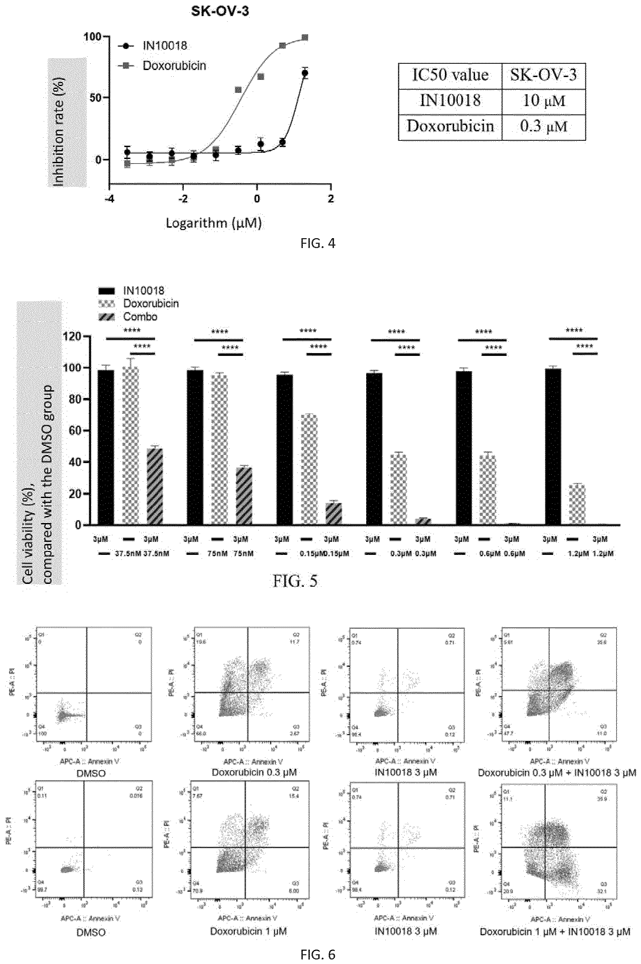

A 96-well cell plate was plated with 5000 SK-OV-3 cells/well. After 24 hours, a drug with a maximum concentration of 20 µM was added and diluted with a 3-fold gradient dilution to set a total of 9 concentrations, with the lowest concentration being 0.3 nM. After the drug and cells were co-incubated for 72 hours, 10 µL of CCK8 solution was added to each well, and the plate was incubated in a 37 °C and 5% carbon dioxide incubator for 2 hours. Then, a microplate reader was used for reading with a set absorbance wavelength of 450 nm. The values read were compared with the DMSO control group and plotted using Graphpad 8.0. The IC₅₀ of IN10018 was 10 (µM), and the IC₅₀ of Adriamycin was 0.3 (µM). The results are shown in FIG. 4.

### 2) Synergistic killing effect of the combination of Adriamycin at different dosages and 3 µM IN10018 on SK-OV-3

A 96-well cell plate was plated with 5000 SK-OV-3 cells/well. After 24 hours, the IC₅₀ value of Adriamycin was used as an initial point, and 3 and 2 sub-dosages were set on the left and right sides, respectively. Adriamycin at these dosages was combined with 3 µM IN10018. After 72 hours of co-culture with the cells, cell viability was detected using CCK8 method. Graphpad 8.0 was used for plotting. The results showed that IN10018 can significantly enhance the cell killing effect of different dosages of Adriamycin on ovarian cancer cells SK-OV-3 (see FIG. 5).

### 3) The combination of Adriamycin and IN10018 significantly enhanced the early and late apoptosis of SK-OV-3

SK-OV-3 cells were treated with 0.3 µM or 1 µM Adriamycin in combination with 3 µM IN10018. After 48 hours, the cells were collected, stained using Annexin V kit, and detected by a flow cytometer. The Q2 quadrant showed the late apoptosis, and the Q3 quadrant showed the early apoptosis. The results showed that compared with the single drug group, the early and late apoptosis were significantly enhanced in the combination group (see FIG. 6).

### 4) The combination of Adriamycin and IN10018 significantly enhanced the release and exposure of Calreticulin, HMGB1 and Annexin A1, the ICD targets of SK-OV-3

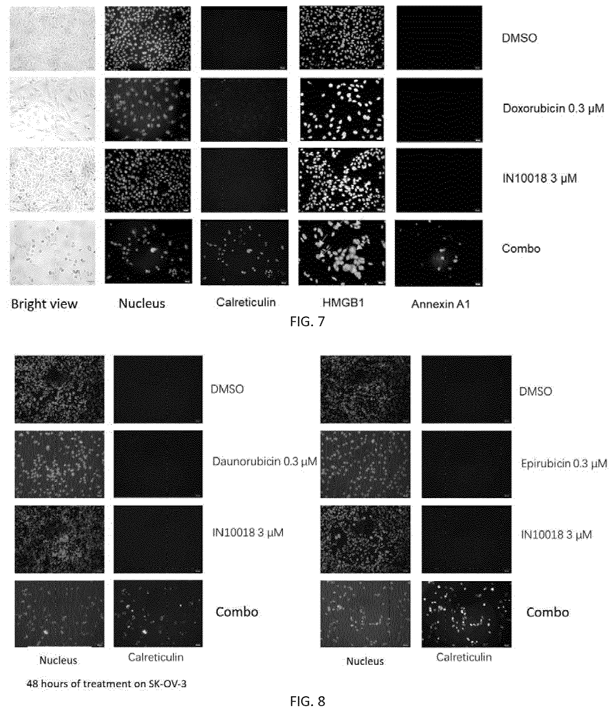

SK-OV-3 cells were treated with 0.3 µM Adriamycin combined with 3 µM IN10018. After 48 hours, antibodies against Calreticulin, HMGB1 and Annexin A1 were used for fluorescent staining. The results showed that compared with the single drug group, the release and exposure of Calreticulin, HMGB1 and Annexin A1 were significantly enhanced in the combination group (see FIG. 7).

### Assay conclusion:

IN10018 can significantly enhance the cell killing effect of Adriamycin on ovarian cancer cells SK-OV-3. This effect is related to the enhancement of early and late apoptosis. In the assay of detecting ICD markers, it was found that IN10018 can significantly enhance the exposure and release of DAMP markers Calreticulin, HMGB1 and Annexin A1, suggesting that IN10018 can effectively enhance the immunogenic cell death of ovarian cancer cells.

### Example 3: Study on the enhancement of immunogenic cell death of ovarian cancer cells in response to daunorubicin and epirubicin by IN10018

### Assay protocol:

IN10018 combined with Daunorubicin or Epirubicin can effectively induce the ICD effect of ovarian cancer cells SK-OV-3

An in vitro assay was conducted to test whether 48 hours of combined use of IN10018 and Daunorubicin or Epirubicin can effectively increase the release of Calreticulin, the main target of ICD, from cells.

### Assay materials:

### 1) Drugs used in this assay

IN10018 was synthesized according to the method in patent WO2010058032. Daunorubicin was provided by MCE. Epirubicin was provided by MCE.

### 2) Antibody used in this assay

Fluorescent Anti-Calreticulin antibody (Abcam, ab196159).

### Assay instrument:

Fluorescence microscope (Olympus U-HGLGPS).

### Assay results:

The combination of Daunorubicin or Epirubicin and IN10018 significantly enhanced the release and exposure of Calreticulin, the ICD target of SK-OV-3.

SK-OV-3 cells were treated with 0.3 µM Daunorubicin or Epirubicin combined with 3 µM IN10018. After 48 hours, a Calreticulin antibody was used for fluorescent staining. The results showed that compared with the single drug group, the release and exposure of Calreticulin were significantly enhanced in the combination group (see FIG. 8).

### Assay conclusion:

In the assay of detecting ICD markers, it was found that IN10018 can significantly enhance the exposure and release of DAMP marker Calreticulin, suggesting that IN10018 can effectively enhance the immunogenic cell death induced by Daunorubicin or Epirubicin.

### Example 4: Study on the enhancement of immunogenic cell death of ovarian cancer cells in response to Adriamycin by Defactinib

### Assay protocol:

### 1) Defactinib combined with Adriamycin produced synergistic tumor killing effect

The cytotoxic effects of Adriamycin at different dosages combined with 3 µM Defactinib were explored.

### 2) Defactinib combined with Adriamycin can effectively induce the ICD effect of ovarian cancer cells SK-OV-3

An in vitro assay was conducted to test whether 48 hours of combined use of Defactinib and Adriamycin can effectively increase the release of Calreticulin, HMGB1 and Annexin A1, the main targets of ICD, from cells.

### Assay materials:

### 1) Drugs used in this assay

Defactinib was provided by DC chemicals. Adriamycin was provided by MCE.

### 2) Antibodies used in this assay

Recombinant Alexa Fluor^{®} 488 Anti-HMGB1 antibody (Abcam, ab195010); Recombinant Alexa Fluor^{®} 647 Anti-Calreticulin antibody (Abcam, ab196159); Annexin A1 rabbit antibody (Cell signaling technology, 32934S); Anti-rabbit IgG (H+L), F(ab')2 Fragment (Alexa Fluor^{®} 488 Conjugate) (Cell signaling technology, 4412).

### 3) Kit used in this assay

CCK8 cell viability reagent (Cellor lab, CX001).

### Assay instrument:

Microplate reader (Molecular devices M3); Fluorescence microscope (Olympus U-HGLGPS).

### Assay results:

### 1) Synergistic killing effect of the combination of Adriamycin at different dosages and 3 µM Defactinib on SK-OV-3

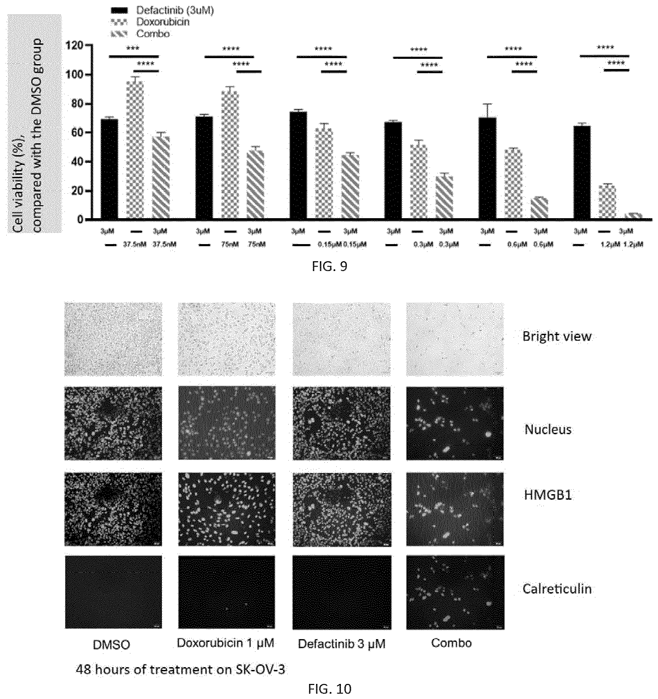

A 96-well cell plate was plated with 5000 SK-OV-3 cells/well. After 24 hours, the IC₅₀ value of Adriamycin was used as an initial point, and 3 and 2 sub-dosages were set on the left and right sides, respectively. Adriamycin at these dosages was combined with 3 µM Defactinib. After 72 hours of co-culture with the cells, cell viability was detected using CCK8 method. Graphpad 8.0 was used for plotting. The results showed that Defactinib can significantly enhance the cell killing effect of different dosages of Adriamycin on ovarian cancer cells SK-OV-3 (see FIG. 9). Student's t-test was used in the statistical research. *** represents the P value is less than 0.005, and **** represents the P value is less than 0.001.

### 2) The combination of Adriamycin and Defactinib significantly enhanced the release and exposure of Calreticulin, HMGB1 and Annexin A1, the ICD targets of SK-OV-3.

SK-OV-3 cells were treated with 1 µM Adriamycin combined with 3 µM Defactinib. After 48 hours, antibodies against Calreticulin, HMGB1 and Annexin A1 were used for fluorescent staining. The results showed that compared with the single drug group, the release and exposure of Calreticulin, HMGB1 and Annexin A1 were significantly enhanced in the combination group (see FIG. 10).

### Assay conclusion:

Defactinib can significantly enhance the cell killing effect of Adriamycin on ovarian cancer cells SK-OV-3. In the assay of detecting ICD markers, it was found that Defactinib can significantly enhance the exposure and release of DAMP markers Calreticulin, HMGB1 and Annexin A1, suggesting that Defactinib can effectively enhance the immunogenic cell death of ovarian cancer cells.

### Example 5: Study on the efficacy of IN10018 and Adriamycin in colon cancer CT26 cell line

### Assay materials:

### 1) Drugs used in this assay

Adriamycin was provided by MCE, Cat No.: HY-15142, Lot No.: 97451.

### 2) Antibodies used in this assay

Recombinant Alexa Fluor^{®} 647 Anti-Calreticulin antibody (Abcam, Cat No.: ab196159, Lot No.: CR33676773); Annexin-Modulation Assay Kit (Beyotime, Cat No.: C1062L, Lot No.: 021921210811).

### Assay method:

CT26 cells were cultured with RPMI 1640 (Shanghai Basalmedia, Cat No.: L210KJ, Lot No.: F210916)+10% FBS (Gibco, Cat No.: 10099-141c, Lot No.: 2158737cp), and passaged twice. When the cells were in good condition, the culture solution was placed in a 24-well plate. After the cells were spread for 24 hours, four groups were set up, wherein the first group was a control group, and the culture medium was added; the second group was IN10018 with a concentration of 5 µM; the third group was Adriamycin (doxorubicin, MCE) with a concentration of 1 µM; and the fourth group was IN10018 (5 µM) combined with Adriamycin (1 µM). The drugs were mixed, and the mixture was cultured in a 5% CO₂ incubator at 37 °C for 48 hours.

### Assay results:

After 48 hours of drug action, the cells were observed with a microscope and photos were taken to confirm the cell killing effect. Then the cells were collected for flow analysis. The cells were washed twice with flow buffer (PBS+2% FBS) and 0.5 µl of AF647. An anti-Calreticulin antibody (abcam) was added to each well and mixed. The mixture was incubated at 4 °C in the dark. After 20 minutes of incubation, the flow buffer was added. An Annexin staining kit (Beyotime) was used, and 195 µl of Annexin-V-FITC conjugate was added and mixed with the cells by pipetting. Then, 5 µl of Annexin-V-FITC antibody was added and gently mixed. Finally, 10 µl of PI dye was added and mixed. The mixture was incubated at room temperature in the dark for 15 min. A sample was sent to a flow cytometer for signal determination.

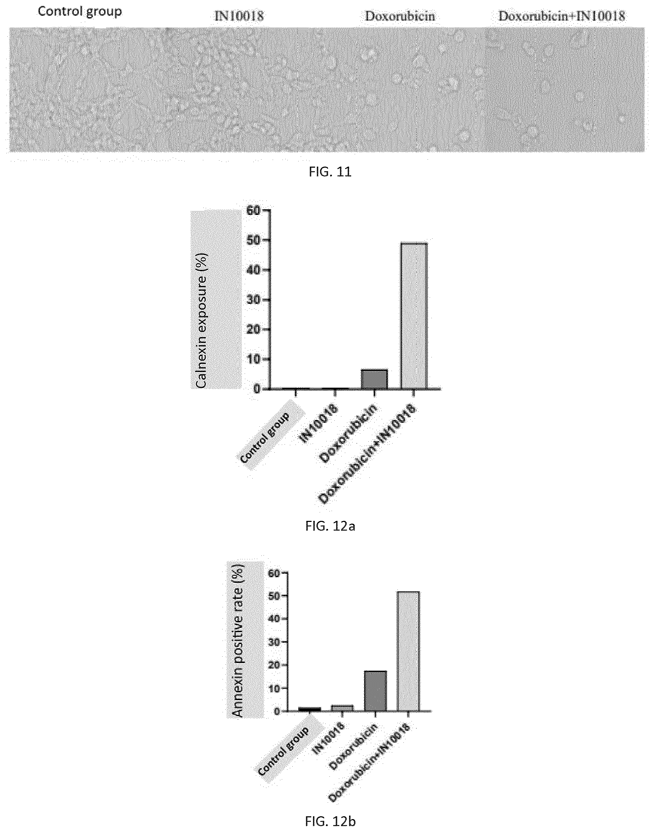

The cells were observed under a microscope. The Adriamycin single-drug group and the two-drug combination group had cell-killing effect. The two-drug combination group had the most significant cell-killing effect, and the cells in the control group and IN10018 group were in good condition, as shown in FIG. 11. The results of flow cytometry analysis showed that the CRT positive rate and Annexin-V positive rate of the two-drug combination group were significantly better than those of the single-drug group and the control group, as shown in FIG. 12a and FIG. 12b.

### Example 6: Study on the in vivo anti-tumor efficacy of IN10018 in subcutaneous allograft tumor model of colon cancer cell CT26 in BALB/c mice

### Assay materials:

Mice: Female BALB/c mice aged 6-8 weeks were purchased from Shanghai Slack Laboratory Animal Co., Ltd.

After the animals arrived, they were raised for acclimatization in the assay environment before the assay. The animals were raised in cages (4 animals per cage) with IVC (independent air supply system) in a SPF animal room. All the cages, padding and drinking water were sterilized before use. All the experimenters wore protective clothing and latex gloves when operating in the animal room. The animal information card for each cage indicated the number, sex, strain, receiving date, administration plan, assay number, group and assay start date of the animals in the cage. Cages, feed and drinking water were replaced twice a week. The feeding environment and illumination condition were as follows:
temperature: 20 to 26 °C; humidity: 40 to 70%;
lighting period: 12 hours of light, 12 hours of dark;
cage: made of polycarbonate, with a volume of 300 mm × 180 mm × 150 mm; the padding was corncob, which was replaced twice a week;
food: assay animals can eat freely during the whole assay (radiation sterilization, dry granular food);
drinking water: assay animals can drink sterilized water freely;
animal identification: assay animals were identified by ear tags.
Information of compound is shown in Table 1

**Table 1. Information of the compound**

| **Drug name** | **Concentration (mg/mL)** | **Storage condition** | **Source** | **Solvent** | **Total amount** |
|---|---|---|---|---|---|
| IN10018 | / | 4 °C | It can be synthesized according to the method in patent WO2010058032 | 0.5% hydroxyethyl cellulose | 350 mg |
| Doxil | 2 mg/mL | 4 °C | Changzhou Jinyuan Pharmaceutical Manufacturing Co., Ltd. | DPBS | 8 mg |
| PD-L1 antibody | 6.60 mg/mL | 4 °C | Bioxcell (BE0101) | DPBS | 25 mg |
| Hydroxyethyl cellulose | / | 4°C | Aladdin | DDH₂O | / |

Colorectal cancer cells CT26 (from the Cell Bank of the Chinese Academy of Sciences, article number: TCM37) were maintained and passaged by Huiyuan Biotechnology (Shanghai) Co., Ltd. The cells were cultured in monolayer in vitro, and the culture conditions were RPMI-1640 medium with 10% fetal bovine serum, 37 °C and 5% CO₂ incubator. Trypsin-EDTA was used for routine digestion and passage twice or three times a week. When the cells were in exponential growth period and the saturation reached 80% to 85%, the cells were collected, counted and inoculated.

### Cell inoculation and grouping

0.1 mL of cell suspension containing 2×10⁵ cells was subcutaneously inoculated on the right back of each mouse. When the tumor volume reached about 38 mm³ (9th day after the cell inoculation), mice were randomly divided into groups according to the tumor volume and administered. The grouping information is shown in Table 2.

**Table 2. Administration scheme of the assay substance in the CT26 allograft tumor model in mice**

| **Group** | **Compound for treatment** | **Numb er of anima ls¹** | **Dosage (mg/kg)** | **Dosage volume parameter (mL/kg)²** | **Route of administr ation** | **Frequency of administration** |
|---|---|---|---|---|---|---|
| 1 | Control group | 8 | N/A | 10 | *p.o.* | QD |
| 2 | IN10018+Doxil | 8 | 12.5+1.5 | 10 | *p.o.*+*i.v.* | QD*28+QW*4 weeks |
| 3 | PD-L1 antibody | 8 | 2.5 | 10 | *i.p.* | BIW*4 weeks |
| 4 | IN10018+Doxil +PD-L1 antibody | 8 | 12.5+1.5+2.5 | 10 | *p.o.*+*i.v.*+ *i.p.* | QD*28+QW*4 weeks+BIW*4 weeks |
| 5 | Doxil+PD-L1 antibody | 8 | 1.5+2.5 | 10 | *i.v.*+*i.p.* | QW*4 weeks+BIW*4 weeks |

| | | | | | | |
|---|---|---|---|---|---|---|
| Note: 1. The number of mice in each group; 2. Dosage volume: 10 mL/kg according to the body weight of mice. If the body weight loss exceeded 15%, the administration to the animal should be stopped; when the body weight returned to a loss of 10%, the administration was resumed. | | | | | | |

### Preparation of assay substance

### See Table 3 for details

**Table 3. Preparation method of the assay substance**

| Drug name | Dosage (mg/kg) | Concentr ation (mg/mL) | Preparation process |
|---|---|---|---|
| Control group (0.5% hydroxyethyl cellulose) | / | / | 0.75g of hydroxyethyl cellulose was weighed, and DDH₂O was added to adjust the volume to 150 mL. The mixture was vortexed to give a clear solution. |
| IN10018 | 12.50 | 1.25 | 15.3 mg of IN10018 was weighed, and 12.24 mL of 0.5% hydroxyethyl cellulose was added for dilution, resulting a suspension after the preparation. |
| Doxil | 1.50 | 0.15 | 0.540 mL of Doxil (2.0 mg/mL) was pipetted. 6.666 mL of DPBS was added for dilution and mixed thoroughly to give a clear solution. |
| PD-L1 antibody | 2.50 | 0.25 | 0.273mL of PD-L1 antibody (6.6 mg/mL) was pipetted. 6.927 mL of DPBS was added for dilution and mixed thoroughly to give a clear solution. |

### Daily observation of the assay animals

The formulation and any modification of this assay protocol were evaluated and approved by the IACUC of Huiyuan Biotechnology. The use and welfare of the assay animals were complied with AAALAC regulations. The health status and death of animals were monitored every day. Observing the effects of tumor growth and drug treatment on the daily behavior of animals, such as behavioral activities, food and water intake (visual inspection only), body weight change, appearance signs, or other abnormal conditions was included in the routine examination. The animals were cared for and assisted with artificial urination every day. Based on the number of animals in each group, the number of dead animals and side effects in the group were recorded.

### Termination of the assay

If the animal's health continues to deteriorate, or the tumor volume exceeds 2,000 mm³, or the animal has a serious disease or pain, the animal must be euthanized. The veterinarian should be notified and the animal should be euthanized if the following situations occur: obvious emaciation, body weight loss greater than 20%; unable to eat and drink freely; the average tumor volume in the control group reaches 2,000 mm³, and the assay will be terminated; the animal exhibits the following clinical manifestations and continues to worsen: piloerection, hunching back, the color of ears, nose, eyes or feet turns to white, shortness of breath, convulsions, continuous diarrhea, dehydration, slow movement and vocalization.

### Tumor measurement and assay index

The diameter of tumor was measured with a vernier caliper 3 times a week. The calculation formula of tumor volume is: V = 0.5 × *a* × *b*², where *a* and *b* represent the long and short diameters of the tumor, respectively.

Referring to the tumor volume on the first day after grouping, the tumor growth inhibition rate TGI (%) was calculated according to the following formula: TGI (%)=[1-(the average tumor volume of an administration group-the average tumor volume of the administration group at the beginning of treatment)/(the average tumor volume of a solvent control group-the average tumor volume of the solvent control group at the beginning of treatment)] × 100%.

### Statistical analysis

Statistical analysis was performed using SPSS 24 software based on the tumor volume and tumor weight at the end of the assay. The comparison between multiple groups was analyzed by one-way ANOVA. If the variances were equal (F > 0.05), Tukey's test was used for analysis; if the variances were unequal (F < 0.05), Games-Howell test was used for analysis. P < 0.05 was considered to have a significant difference.

### Assay results

The in vivo efficacy of the assay substance IN10018 and/or PD-L1 antibody in combination with the positive drug Doxil in the subcutaneous allograft tumor model of mouse colorectal cancer cell CT26 in BALB/c mice. After the cell inoculation, the tumor growth was observed every day, and the animals were divided into groups according to the tumor volume on the 9th day after inoculation. The average tumor volume of the enrolled mice was about 38 mm³. Due to the tumor load, mice in the control group (G1) were euthanized on day 28, and mice in the PD-L1 antibody group (G3) #790 were euthanized on day 32. The whole assay was ended on day 35.

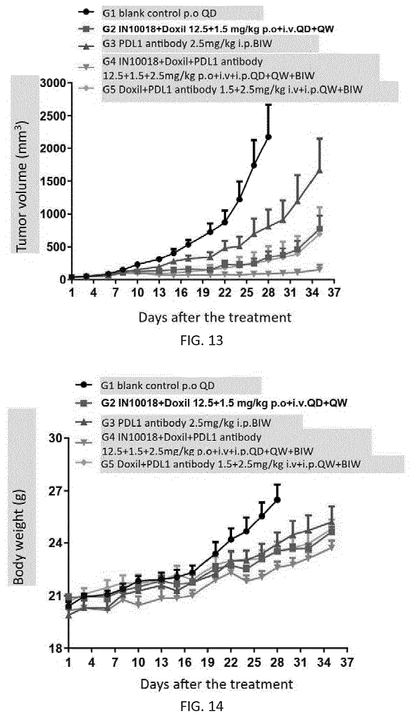

On the 28th day after group administration, the tumor volume of the control group was 2176.53±491.44 mm³. The tumor volumes of the IN10018+Doxil (12.5+1.5 mg/kg), PD-L1 antibody (2.5 mg/kg), IN10018+Doxil+PD-L1 antibody (12.5+1.5+2.5 mg/kg) and Doxil+PD-L1 antibody (1.5+2.5 mg/kg) treatment groups were 340.51±89.1 mm³, 807.22±257.97 mm³, 88.95±33.38 mm³ and 295.53±215.08 mm³, respectively. The details are shown in Table 4. Comparing the comprehensive tumor volume, the tumor growth inhibition rates (TGI) of IN10018+Doxil (12.5+1.5 mg/kg) group, PD-L1 antibody (2.5 mg/kg) group, IN10018+Doxil+PD-L1 antibody (12.5+1.5+2.5 mg/kg) group and Doxil+PD-L1 antibody (1.5+2.5 mg/kg) group were 85.91% *(p =* 0.041), 64.05% *(p* = 0.171), 97.66% *(p* = 0.022) and 88.00% *(p* = 0.038), respectively. The details are shown in Table 4. The tumor volumes of each dosage group at different time periods are shown in FIG. 13.

**Table 4 Evaluation of the tumor inhibition effect of the assay substance on the allograft tumor model of mouse colon cancer cell CT26 in BALB/c mice (based on the data of day 28)**

| **Group** | **Tumor volume on day 1 (mm³)¹** | **Tumor volume on day 25 (mm³)** | **TGI(%)** |
|---|---|---|---|
| Control group | 38.86±1.17 | 2176.53±491.44 | / |
| IN10018 + Doxil | 39.38±1.27 | 340.51±89.1 | 85.91 |
| PD-L1 antibody | 38.74±1.4 | 807.22±257.97 | 64.05 |
| IN10018 + Doxil + PD-L1 antibody | 38.94±1.44 | 88.95±33.38 | 97.66 |
| Doxil + PD-L1 antibody | 38.99±1.28 | 295.53±215.08 | 88.00 |

| | | | |
|---|---|---|---|
| Note: The data is mean±standard error of the mean. | | | |

The assay was conducted according to the administration scheme. During the assay, animal activities such as eating and drinking were observed every day, and the animal body weight was recorded 3 times a week. The body weight curves of the animals are shown in FIG. 14. During the whole administration cycle, the animals in each group showed no significant weight loss and were in good condition.

### Conclusion

Compared with the blank control group, each of the IN10018+Doxil (12.5+1.5 mg/kg), PD-L1 antibody (2.5 mg/kg), IN10018+Doxil+PD-L1 antibody (12.5+1.5+2.5 mg/kg) and Doxil+PD-L1 antibody (1.5+2.5 mg/kg) treatment groups had a significant tumor growth inhibition effect, and a tumor growth inhibition rate (TGI) greater than 60%. The IN10018+Doxil+PD-L1 antibody (12.5+1.5+2.5 mg/kg) group had the best tumor inhibition effect.

### Example 7: Study on the in vivo anti-tumor efficacy of Defactinib in subcutaneous allograft tumor model of colon cancer cell CT26 in BALB/c mice

### Assay materials:

Mice: Female BALB/c mice aged 6-8 weeks were purchased from Beijing Vital River Laboratory Animal Technology Co., Ltd. After the animals arrived, they were raised for acclimatization in the assay environment before the assay. The animals were raised in cages (5 animals per cage) with IVC (independent air supply system) in a SPF animal room. All the cages, padding and drinking water were sterilized before use. All the experimenters wore protective clothing and latex gloves when operating in the animal room. The animal information card for each cage indicated the number, sex, strain, receiving date, administration plan, assay number, group and assay start date of the animals in the cage. Cages, feed and drinking water were replaced twice a week. The feeding environment and illumination condition were as follows:
temperature: 20 to 26 °C; humidity: 40 to 70%;
lighting period: 12 hours of light, 12 hours of dark;
cage: made of polycarbonate, with a volume of 300 mm × 180 mm × 150 mm; the padding was corncob, which was replaced twice a week;
food: assay animals can eat freely during the whole assay (radiation sterilization, dry granular food);
drinking water: assay animals can drink sterilized water freely;
animal identification: assay animals were identified by ear tags.
Information of compound is shown in Table 5.

**Table 5. Information of the compound**

| Drug name | Concentrati on (mg/mL) | Storage conditi on | Source | Solvent | Total amount |
|---|---|---|---|---|---|
| Defactinib | / | Room temper ature | DC chemicals (DC7695) | 0.5% cellulose hydroxyethyl ether | 320 mg |
| Doxil | 2 mg/mL | 4 °C | Changzhou Jinyuan Pharmaceutical Manufacturing Co., Ltd. | DPBS | 4 mg |
| PD-L1 antibody | 8.13 mg/mL | 4 °C | Bioxcell (BE0101) | DPBS | 40 mg |
| Cellulose hydroxyethyl ether | / | Room temper ature | Aladdin | DDH₂O | / |

Colorectal cancer cells CT26 (from Nanjing Kebai Biotechnology Co., Ltd., article number: CBP60043) were maintained and passaged by InxMed (Nanjing) Co., Ltd. The cells were cultured in monolayer in vitro, and the culture conditions were RPMI-1640 medium with 10% fetal bovine serum, 37 °C and 5% CO₂ incubator. Trypsin-EDTA was used for routine digestion and passage twice or three times a week. When the cells were in exponential growth period and the saturation reached 80% to 90%, the cells were collected, counted and inoculated.

### Cell inoculation and grouping

0.1 mL of cell suspension containing 3×10⁵ cells was subcutaneously inoculated on the right back of each mouse. When the tumor volume reached about 81 mm³ (9th day after the cell inoculation), mice were randomly divided into groups according to the tumor volume and administered. The grouping information is shown in Table 6.

**Table 6. Administration scheme of the assay substance in CT26 allograft tumor model in mice**

| **Gr on P** | **Compound for treatment** | **Num ber of anim als¹** | **Dosage (mg/kg)** | **Dosage volume parameter (mL/kg)²** | **Route of administ ration** | **Frequency of administration** |
|---|---|---|---|---|---|---|
| 1 | Control group | 7 | N/A | 10 | *p.o.* | BID |
| 2 | Defactinib+Doxil | 7 | 25+1.5 | 10 | *p.o.*+*i.v.* | BID*15+QW*2 weeks |
| 3 | Doxil | 7 | 1.5 | 10 | *i.v.* | QW*2 weeks |
| 4 | PD-L1 antibody | 7 | 10 | 10 | *i.p.* | BIW*2 weeks |
| 5 | Doxil+PD-L1 antibody | 7 | 1.5+10 | 10 | *i.v.*+*i.p.* | QW*2 weeks+BIW*2 weeks |
| 6 | Defactinib+Doxil +PD-L1 antibody | 7 | 25+1.5+10 | 10 | *p.o.*+*i.v.* +*i.p.* | BID*15+QW*2 weeks+BIW*2 weeks |

| | | | | | | |
|---|---|---|---|---|---|---|
| Note: 1. The number of mice in each group; 2. Dosage volume: 10 mL/kg according to the body weight of mice. If the body weight loss exceeded 15%, the administration to the animal should be stopped; when the body weight returned to a loss of 10%, the administration was resumed. | | | | | | |

### Preparation of assay substance

### See Table 7 for details

**Table 7. Preparation method of the assay substance**

| **Drug name** | **Dosage (mg/kg)** | **Concent ration (mg/mL)** | **Preparation process** |
|---|---|---|---|
| **Control group (0.5% cellulose hydroxyethyl ether)** | / | / | 0.75g of cellulose hydroxyethyl ether was weighed, and DDH₂O was added to adjust the volume to 150 mL. The mixture was vortexed to give a clear solution. |
| **Defactinib** | 25.00 | 2.5 | 84.0 mg of Defactinib was weighed, and 33.6 mL of 0.5% cellulose hydroxyethyl ether was added for dilution, resulting a suspension after the preparation. The suspension was prepared every 4 days. |
| **Doxil** | 1.50 | 0.15 | 0.65 mL of Doxil (2.0 mg/mL) was pipetted. 8.017 mL of DPBS was added for dilution and mixed thoroughly to give a clear solution. The solution was prepared before each use. |
| **PD-L1 antibody** | 10.00 | 1.00 | 0.775 mL of PD-L1 antibody (8.13 mg/mL) was pipetted. 5.525 mL of DPBS was added for dilution and mixed thoroughly to give a clear solution. The solution was prepared before each use. |

### Daily observation of the assay animals

The formulation and any modification of this assay protocol were evaluated and approved by the IACUC of Array Bridge. The use and welfare of the assay animals were complied with AAALAC regulations. The health status and death of animals were monitored every day. Observing the effects of tumor growth and drug treatment on the daily behavior of animals, such as behavioral activities, food and water intake (visual inspection only), body weight change, appearance signs, or other abnormal conditions was included in the routine examination. Based on the number of animals in each group, the number of dead animals and side effects in the group were recorded.

### Termination of the assay

If the animal's health continues to deteriorate, or the tumor volume exceeds 4,000 mm³, or the animal has a serious disease or pain, the animal must be euthanized. The veterinarian should be notified and the animal should be euthanized if the following situations occur: obvious emaciation, body weight loss greater than 20%; unable to eat and drink freely; the average tumor volume in the control group reaches 4,000 mm³, and the assay will be terminated; the animal exhibits the following clinical manifestations and continues to worsen: piloerection, hunching back, the color of ears, nose, eyes or feet turns to white, shortness of breath, convulsions, continuous diarrhea, dehydration, slow movement and vocalization.

### Tumor measurement and assay index

The diameter of the tumor was measured with a vernier caliper 3 times a week. The calculation formula of the tumor volume is: V = 0.5 × *a* × b², where *a* and *b* represent the long and short diameters of the tumor, respectively.

Referring to the tumor volume on the first day after grouping, the tumor growth inhibition rate TGI (%) was calculated according to the following formula: TGI (%)=[1-(the average tumor volume of an administration group-the average tumor volume of the administration group at the beginning of treatment)/(the average tumor volume of a solvent control group-the average tumor volume of the solvent control group at the beginning of treatment)] × 100%.

### Statistical analysis

Statistical analysis was performed using Prism Graphpad software based on the tumor volume and tumor weight at the end of the assay. The comparison between multiple groups was analyzed by Two-way ANOVA. P < 0.05 was considered to have a significant difference.

### Assay results

The in vivo efficacy of the assay substance Defactinib and/or PD-L1 antibody in combination with the positive drug Doxil in the subcutaneous allograft tumor model of mouse colorectal cancer cell CT26 in BALB/c mice. After the cell inoculation, the tumor growth was observed every day, and the animals were divided into groups according to the tumor volume on the 9th day after inoculation. The average tumor volume of the enrolled mice was about 81 mm³. Due to the tumor load, all the enrolled mice were euthanized on the 24th day after inoculation, that is, the 15th day after group administration, and the whole assay was ended.

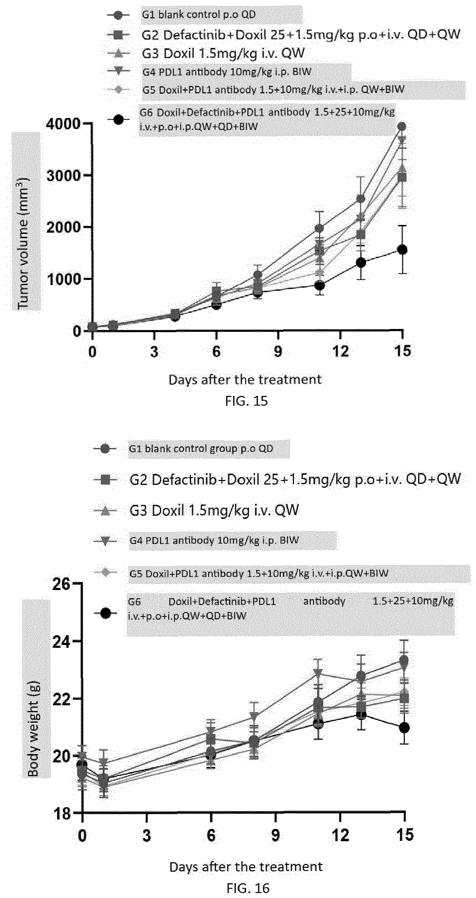

On the 15th day after group administration, the tumor volume of the control group was 3920.2±1697.5 mm³. The tumor volumes of the Defactinib+Doxil (25+1.5 mg/kg), Doxil single drug (1.5mg/kg), PD-L1 antibody (10 mg/kg), Doxil+PD-L1 antibody (1.5+10 mg/kg) and Defactinib+Doxil+PD-L1 antibody (25+1.5+10 mg/kg) treatment groups were 2942.2±1491.2 mm³, 3130.9±1446.2 mm³, 3645.2±1914.8 mm³, 2964.6±1653.2 mm³ and 1546.5±1237.9 mm³, respectively. The details are shown in Table 8. Comparing the comprehensive tumor volume with the control group, the tumor growth inhibition rates (TGI) of Defactinib+Doxil (25+1.5 mg/kg) group, Doxil single drug (1.5mg/kg) group, PD-L1 antibody (10 mg/kg) group, Doxil+PD-L1 antibody (1.5+10 mg/kg) group and Defactinib+Doxil+PD-L1 antibody (25+1.5+10 mg/kg) group were 25.49% (p = 0.0470), 20.55% *(p =* 0.1513), 7.17% *(p* = 0.9267), 24.89% *(p* = 0.0547) and 61.83% *(p <* 0.0001), respectively. Comparing the comprehensive tumor volume with the Defactinib+Doxil+PD-L1 antibody (25+1.5+10 mg/kg) group for statistical analysis, the P values of the control group, Defactinib+Doxil (25+1.5 mg/kg) group, Doxil single drug (1.5mg/kg) group, PD-L1 antibody (10 mg/kg) group and Doxil+PD-L1 antibody (1.5+10 mg/kg) group were p *<* 0.0001, *p =* 0.0016, *p =* 0.0002, *p <* 0.0001 *and p =* 0.0013, respectively. The details are shown in Table 8. The tumor volumes of each dosage group at different time periods are shown in FIG. 15.

**Table 8 Evaluation of the tumor inhibition effect of the assay substance on the allograft tumor model of mouse colon cancer cell CT26 in BALB/c mice (based on the data on the 15th day after group administration)**

| **Group** | **Tumor volume on day 0 (mm³)¹** | **Tumor volume on day 15 (mm³)** | **TGI (%)** | **P value²** | **P value³** |
|---|---|---|---|---|---|
| **Control group** | 81.0±31.9 | 3920.2±1697.5 | / | / | <0.0001 **** |
| **Defactinib+ Doxil** | 81.8±30.3 | 2942.2±1491.2 | 25.49 | 0.0470 * | 0.0016 ** |
| **Doxil single drug** | 80.8±40.8 | 3130.9±1446.2 | 20.55 | 0.1513 | 0.0002 *** |
| **PD-L1 antibody** | 81.3±36.3 | 3645.2±1914.8 | 7.17 | 0.9267 | <0.0001**** |
| **Doxil+PD-L1 antibody** | 81.1±35.9 | 2964.6±1653.2 | 24.89 | 0.0547 | 0.0013 ** |
| **Defactinib+Doxil+ PD-L1 antibody** | 81.0±29.1 | 1546.5±1237.9 | 61.83 | <0.0001**** | / |

| | | | | | |
|---|---|---|---|---|---|
| Note: 1. The results were calculated according to the days after group administration, and the data is mean±standard error of the mean. 2. *: p<0.05, ****: p<0.0001, vs. control group, Two-way ANOVA. 3. **: p<0.01, ***: p<0.001, ****: p<0.0001, vs. Defactinib+Doxil+PD-L1 antibody (25+1.5+10 mg/kg) group, Two-way ANOVA. | | | | | |

The assay was conducted according to the administration scheme. During the assay, animal activities such as eating and drinking were observed every day, and the animal body weight was recorded 3 times a week. The body weight curves of the animals were shown in FIG. 16. During the whole administration cycle, the animals in each group showed no significant weight loss and were in good condition.

### Conclusion

Compared with the blank control group, both of the Defactinib+Doxil (25+1.5 mg/kg) group and the Defactinib+Doxil+PD-L1 antibody (25+1.5+10 mg/kg) group had significant tumor growth inhibition effect, and had statistical differences compared with the control group. The Defactinib+Doxil+PD-L1 antibody (25+1.5+10 mg/kg) group had smaller tumors compared with the Defactinib+Doxil (25+1.5 mg/kg), Doxil single drug (1.5mg/kg), PD-L1 antibody (10 mg /kg) and Doxil+PD-L1 antibody (1.5+10 mg/kg) treatment groups, and there were statistical differences compared with these single-drug groups and two-drug combination groups. Compared with the single-drug groups and the two-drug combination groups, the Defactinib+Doxil+PD-L1 antibody (25+1.5+10 mg/kg) group had better tumor growth inhibition effect.

### Example 8: ELISA detection of IFN-γ and Granzyme B in the serum of subcutaneous allograft tumor model of colon cancer cell CT26 in BALB/c mice after the combined treatment of assay substances Doxil and IN10018

### Assay materials

The assay samples are shown in Table 9.

**Table 9:**

| Name | Doxorubicin hydrochloride liposome injection | IN10018 |
|---|---|---|
| Code name | Doxil | IN10018 |
| Supplier | Changzhou Jinyuan Pharmaceutical Manufacturing Co., Ltd. | It can be synthesized according to the method in patent WO2010058032 |
| Concentration | 10ml:20mg | |
| Property | Red viscous liquid | White powder |
| Solvent | Special solvent & PBS | DPBS |
| Storage condition | 4 °C | Room temperature |
| Amount of usage this time | 10mg | 190mg |

Information of the assay reagents was shown in Table 10.

**Table 10:**

| Name | Brand | Article number | Lot number |
|---|---|---|---|
| Mouse IFN-γ ELISA Kit | Beyotime | P1507 | 051922220705 |
| Mouse Granzyme B ELISA Kit | Boster | EK1115 | 66117109707 |

### Assay method and procedure

BALB/c mice (from Shanghai Lingchang Biotechnology Co., Ltd.) were used to construct the CT26 tumor cell model. When the tumor volume reached about 40 to 60 mm³, the mice were randomly divided into groups according to the tumor volume and administered. The grouping information is shown in Table 11.

**Table 11: Grouping of the assay animals and administration scheme**

| Group | N¹ | Assay drug | Dosage (mg/kg) | Dosage volume parameter (mL/kg)² | Route of administrati on | Frequency of administration |
|---|---|---|---|---|---|---|
| G1 | 5 | Blank control | N/A | 10 | PO | QD × 8 |
| G2 | 5 | IN10018 | 25 | 10 | PO | QD × 8 |
| G3 | 5 | Doxil | 3 | 10 | i.v. | QW × 2 |
| G4 | 5 | Doxil+IN10018 | 3+25 | 10 | i.v.+PO | QW × 2+ QD × 8 |

| | | | | | | |
|---|---|---|---|---|---|---|
| Note: 1. N: The number of mice in each group 2. Dosage volume: 10 mL/kg according to the body weight of mice. If the body weight loss exceeded 15%, the administration to the animal should be stopped; when the body weight returned to a loss of 10%, the administration was resumed. | | | | | | |

### Collection and preparation of mouse serum

24 hours after the second administration of Doxil, blood was collected from the orbit of four groups of animals. The blood was placed in a collection tube without anticoagulant and left standing to naturally coagulate at room temperature for 30 to 60 minutes. When the blood coagulated, the blood was centrifuged at 1000 rpm, 4 °C for 10 minutes. The upper serum was transferred to a new collection tube and stored in a -80 °C freezer.

### Assay of IFN-γ in mouse serum

The sample was added to a corresponding well at 100 µl/well (the sample was diluted 5-fold with a sample diluent in advance), and the reaction well was sealed with a sealing film (transparent) and incubated at room temperature for 120 minutes; the plate was washed 5 times, and patted dry on a thick absorbent paper after the last wash; a biotinylated antibody was added at 100 µl/well, and the reaction well was sealed with a sealing film (transparent) and incubated at room temperature for 60 minutes; the plate was washed 5 times, and patted dry on a thick absorbent paper after the last wash; horseradish peroxidase-labeled Streptavidin was added at 100 µl/well, and the reaction well was sealed with a sealing film (white) and incubated at room temperature in the dark for 20 minutes; when the room temperature was low (below 25 °C), the incubation time was in need to be appropriately extended; the plate was washed 5 times, and patted dry on a thick absorbent paper after the last wash; TMB chromogen solution was added at 100 µl/well, and the reaction well was sealed with a sealing film (white) and incubated at room temperature in the dark for 15 to 20 minutes; when the room temperature was low, the incubation time was in need to be appropriately extended until the standard showed a very significant color change; if the sample concentration was high enough, the sample would also show a significant color change; stop solution was added at 50 µl/well and mixed well, and A450 values were measured immediately.

### Assay of Granzyme B in mouse serum

The sample was added to a corresponding well at 100 µl/well (the sample was diluted 5-fold with a sample diluent in advance), and the reaction well was sealed with a sealing film (transparent), and incubated at 37 °C for 90 minutes without washing; a biotinylated antibody was added at 100 µl/well, and the reaction well was sealed with a sealing film (transparent), and incubated at 37 °C for 60 minutes; the plate was washed 3 times and patted dry on a thick absorbent paper after the last wash; Avidin-Biotin-Peroxidase Complex (ABC) was added at 100 µl/well, and the reaction well was sealed with a sealing film (white) and incubated at 37 °C in the dark for 30 minutes; the plate was washed 5 times, and patted dry on a thick absorbent paper after the last wash; TMB chromogen solution was added at 90 µl/well, and the reaction well was sealed with a sealing film (white) and incubated at room temperature in the dark for 15 to 20 minutes; when the room temperature was low, the incubation time was in need to be appropriately extended until the standard showed a very significant color change; if the sample concentration was high enough, the sample would also show a significant color change; stop solution was added at 100 µl/well and mixed well, and A450 values were measured immediately.

### Statistical analysis

Statistical analysis was performed using Prism Graphpad software. The comparison between multiple groups was analyzed by Two-way ANOVA and Kruskal-Wallis test. P < 0.05 was considered to have a significant difference.

### Assay results

### Assay results of IFN-γ in mouse serum

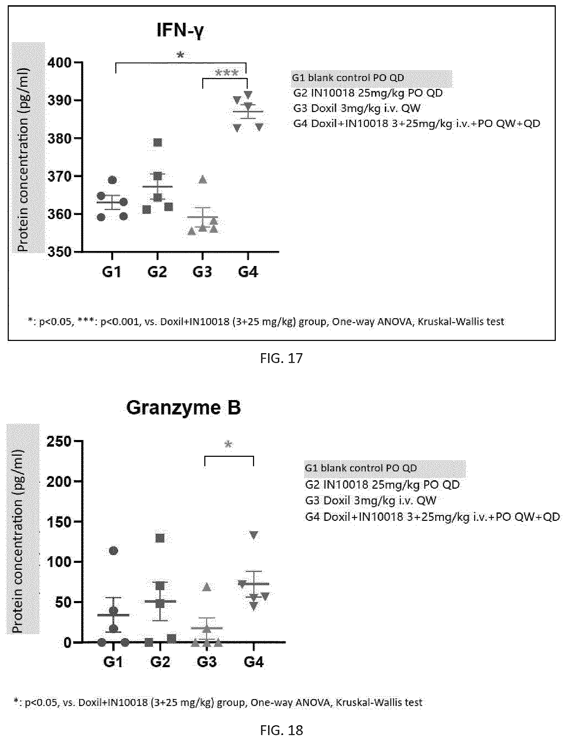

The IFN-γ protein concentrations of blank control group, IN10018 group, Doxil group and Doxil+IN10018 group were 363.10±4.06 pg/ml, 367.24±7.34 pg/ml, 359.13±5.71 pg/ml, and 387.00±4.04 pg/ml, respectively. Comparing the comprehensive protein concentration with the Doxil+IN10018 group, the Doxil+IN10018 two-drug combination group had higher protein expression than the Doxil single-drug group. Statistical analysis was performed, and P value was p < 0.001. The details are shown in FIG. 17.

### Assay results of Granzyme B in mouse serum

The Granzyme B protein concentrations of blank control group, IN10018 group, Doxil group, and Doxil+IN10018 group were 34.06±47.49 pg/ml, 50.48±53.08 pg/ml, 17.24±30.80 pg/ml, and 72.21±35.40 pg/ml, respectively. Comparing the comprehensive protein concentration with the Doxil+IN10018 group, the Doxil+IN10018 two-drug combination group had higher protein expression than the Doxil single-drug group. Statistical analysis was performed, and the P value was p < 0.05. The details are shown in FIG. 18.

### Assay conclusion

In the detection assay of the IFN-γ protein concentration in mouse serum, each group was compared with the Doxil+IN10018 group. The Doxil+IN10018 two-drug combination group had higher protein expression than the Doxil single-drug group. Statistical analysis was performed, and the P value was p < 0.001.

In the detection assay of the Granzyme B protein concentration in mouse serum, each group was compared with the Doxil+IN10018 group. The Doxil+IN10018 two-drug combination group had higher protein expression than the Doxil single-drug group. Statistical analysis was performed, and the P value was p < 0.05.

### Example 9: Study on the induction of immunogenic cell death targets of mouse breast cancer 4T1 cells in vitro by assay compounds Adriamycin and AMP945

### Assay materials:

### 1) Drugs used in this assay

Adriamycin was provided by MCE, Lot No.: 97451

AMP945 was provided by MCE, Lot No.: 143253

### 2) Antibodies used in this assay

Recombinant Alexa Fluor^{®} 647 Anti-Calreticulin antibody (Abcam, Cat No.: ab196159, Lot No.: CR33676773); Annexin V-apoptosis detection kit (Beyotime, Cat No.: C1062L, Lot No.: 122221220706).

### Assay method:

4T1 cells (from Nanjing Kebai Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp). The culture conditions were 37 °C and 5% CO₂. Trypsin was used for routine digestion and passage treatment twice or three times a week. When the cells were in exponential growth period and the confluency of the adherent cells reached 80% to 90%, the cells were collected and plated. The 4T1 cells were digested with trypsin, and then collected and counted. According to the counting results, the cells were diluted with RPMI1640+10%FBS, and the dilution concentration was 50,000 cells/ml. Then, the cells were plated on a 12-well cell culture plate, and 2 ml of cell suspension (100,000 cells) was plated in each well. After the plating, cells were cultured in a 37 °C and 5% CO₂ incubator. 24 hours after the cells were spread, six groups were set up, wherein the first group was a control group, and the culture medium was added; the second group was AMP945 with a concentration of 3µM; the third group was AMP945 with a concentration of 6 µM; the fourth group was Adriamycin (doxorubicin, MCE) with a concentration of 0.5 µM; the fifth group was AMP945 (3 µM) combined with Adriamycin (0.5 µM); and the sixth group was AMP945(6 µM) combined with Adriamycin (0.5 µM). The drugs were mixed, and the mixture was cultured in a 5%CO₂ incubator at 37 °C for 48 hours.

### Assay results

After 48 hours of drug action, the cells were collected for flow analysis. The cells were washed twice with flow buffer (PBS+2%FBS) and 0.5 µl of AF647. An anti-Calreticulin antibody (abcam) was added to each well and mixed. The mixture was incubated at 4 °C in the dark. After 20 minutes of incubation, the flow buffer was added. An Annexin staining kit (Beyotime) was used, and 195 µl of Annexin-V-FITC conjugate was added and mixed with the cells by pipetting. Then, 5 µl of Annexin-V-FITC antibody was added and gently mixed. Finally, 10 µl of PI dye was added and mixed. The mixture was incubated at room temperature in the dark for 15 min. A sample was sent to a flow cytometer for signal determination.

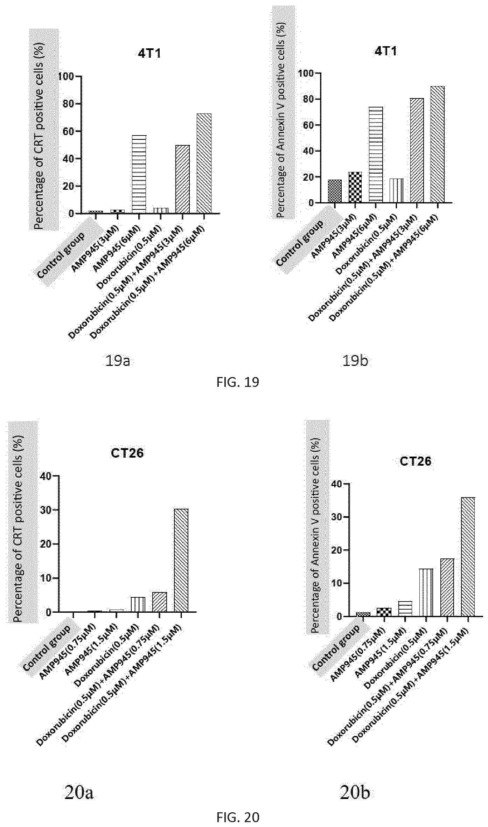

The results of flow cytometry analysis showed that the CRT positive rate and Annexin-V positive rate of the two-drug combination groups were significantly better than those of the single-drug groups and the control group, which are shown in FIG. 19a and FIG. 19b.

### Example 10: Study on the induction of immunogenic cell death targets in mouse colorectal cancer CT26 cells in vitro by assay compounds Adriamycin and AMP945

### Assay materials:

### 1) Drugs used in this assay

Adriamycin was provided by MCE, Lot No.: 97451

AMP945 was provided by MCE, Lot No.: 143253

### 2) Antibodies used in this assay

Recombinant Alexa Fluor^{®} 647 Anti-Calreticulin antibody (Abcam, Cat No.: ab196159, Lot No.: CR33676773); Annexin V-apoptosis detection kit (Beyotime, Cat No.: C1062L, Lot No.: 122221220706).

### Assay method:

CT26 cells (from Nanjing Kebai Biotechnology Co., Ltd., article number: CBP60043) were cultured with RPMI 1640 (Shanghai Basalmedia, Cat No.: L210KJ, Lot No.: F210916)+10% FBS (Gibco, Cat No.: 10099-141c, Lot No.: 2158737cp). The culture conditions were 37 °C and 5% CO₂. Trypsin was used for routine digestion and passage treatment twice or three times a week. When the cells were in exponential growth period and the confluency of the adherent cells reached 80% to 90%, the cells were collected and plated. The CT26 cells were digested with trypsin, and then collected and counted. According to the counting results, the cells were diluted with RPMI1640+10%FBS, and the dilution concentration was 50,000 cells/ml. Then, the cells were plated on a 12-well cell culture plate, and 2 ml of cell suspension (100,000 cells) was plated in each well. After the plating, cells were cultured in a 37 °C and 5% CO₂ incubator. 24 hours after the cells were spread, six groups were set up, wherein the first group was a control group, and the culture medium was added; the second group was AMP945 with a concentration of 0.75 µM; the third group was AMP945 with a concentration of 1.5 µM; the fourth group was Adriamycin (doxorubicin, MCE) with a concentration of 0.5 µM; the fifth group was AMP945 (0.75 µM) combined with Adriamycin (0.5 µM); the sixth group was AMP945 (1.5 µM) combined with Adriamycin (0.5 µM). The drugs were mixed, and the mixture was cultured in a 5%CO₂ incubator at 37 °C for 48 hours.

### Assay results

After 48 hours of drug action, the cells were collected for flow analysis. The cells were washed twice with flow buffer (PBS+2%FBS) and 0.5 µl of AF647. An anti-Calreticulin antibody (abcam) was added to each well and mixed. The mixture was incubated at 4°C in the dark. After 20 minutes of incubation, the flow buffer was added. An Annexin staining kit (Beyotime) was used, and 195 µl of Annexin-V-FITC conjugate was added and mixed with the cells by pipetting. Then, 5 µl of Annexin-V-FITC antibody was added and gently mixed. Finally, 10 µl of PI dye was added and mixed. The mixture was incubated at room temperature in the dark for 15 min. A sample was sent to a flow cytometer for signal determination.

The results of flow cytometry analysis showed that the CRT positive rate and Annexin-V positive rate of the two-drug combination groups were significantly better than those of the single-drug groups and the control group, which are shown in FIG. 20a and FIG. 20b.

### Example 11: Clinical study of IN10018 combined with PLD and anti-PD-1 in the treatment of ovarian cancer

IN10018, in combination with pegylated liposomal doxorubicin (PLD) and anti-PD-1 antibody, for the treatment of platinum-resistant recurrent ovarian cancer was currently evaluated in a Phase Ib/II clinical study (IN10018-010) in China. The IN10018 being used was a tartrate thereof, the PLD being used was a commercially available doxorubicin hydrochloride liposome injection (common name), and the anti-PD-1 antibody being used was a commercially available toripalimab. The subjects of the study will receive 100 mg of IN10018 orally once daily (QD) in combination with 40 mg/m² of PLD every 4 weeks (Q4W) and 3 mg/kg of toripalimab every 2 weeks (Q2W) by intravenous infusion. One treatment cycle was 28 days.

As of November 30, 2022, a total of 41 patients with various solid tumors had been enrolled in the study and received a three-drug combination (IN10018+PLD+anti-PD-1) treatment. Among the 41 patients, 24 patients were with platinum-resistant recurrent ovarian cancer. All these 41 patients received drug treatment at least once, and were included in the safety analysis. Among the enrolled 24 patients with platinum-resistant recurrent ovarian cancer, 13 patients had the first tumor imaging evaluation data and were included in the efficacy analysis.

The baseline characteristics of the enrolled 24 patients with platinum-resistant recurrent ovarian cancer are summarized in Table 12.

**Table 12: Summary of the baseline characteristics of the 26 patients with platinum-resistant recurrent ovarian cancer in the ongoing study**

| **Baseline characteristics** | | **IN10018+PLD+anti-PD-1 (n=24)** | |
|---|---|---|---|
| | | **n** | **%** |
| | **Age (years)** | | |
| | Median (range) | **58 years old (50 to 73 years old)** | |
| | **FIGO staging** | | |
| | III | **16** | **66.7%** |
| | IV | **8** | **33.3%** |
| | **ECOG** | | |
| | 0 | **13** | **54.2%** |
| | 1 | **11** | **45.8%** |
| | **Disease type** | | |
| | Ovarian cancer | **21** | **87.5%** |
| | Primary peritoneal cancer | **2** | **8.3%** |
| | Fallopian tube cancer | **1** | **4.2%** |
| | **Tissue type** | | |
| | High-grade serous carcinoma | **21** | **87.5%** |
| | Mucinous carcinoma | **2** | **8.3%** |
| | Clear-cell carcinoma | **1** | **4.2%** |
| | **Previous use of bevacizumab** | | |
| | Yes | **7** | **29.2%** |
| **Previous use of PARP inhibitor** | | | |
| | Yes | **2** | **8.3%** |

### Efficacy

Changes in tumor load over time were evaluated based on the tumor imaging examination in the study to evaluate the anti-tumor efficacy of the three-drug combination (IN10018+PLD+anti-PD-1) treatment in the subjects with platinum-resistant recurrent ovarian cancer. The first choice of the study was to use the contrast-enhanced computed tomography (CT) for imaging examination. For abdomen and pelvis, the contrast-enhanced magnetic resonance imaging (MRI) can be used when the use of CT with iodinated contrast media was contraindicated. MRI was the first choice for brain imaging, but other imaging techniques such as PET/CT and bone scans can also be used if needed.

Imaging examination during the screening period must be performed within 28 days before the treatment assignment of the study. During the study, the subject will receive a tumor imaging examination every 8 weeks (56±7 days) until an imaging-confirmed disease progression is found through an assessement of the subject by the researcher, or the subject starts a new anti-tumor treatment, withdraws from the study, or dies (whichever occurs first).

The anti-tumor efficacy was evaluated based on the Response Evaluation Criteria in Solid Tumors version 1.1 (RECIST 1.1) in the study. Efficacy indicators included objective response rate (ORR), disease control rate (DCR), duration of response (DOR), progression free survival (PFS) and overall survival (OS).

There were 13 patients with evaluable efficacy in the study of three-drug combination (IN10018+PLD+anti-PD-1) treatment in platinum-resistant recurrent ovarian cancer. Most of the patients only had the evaluation results of the first tumor imaging, so only the efficacy data of the first tumor evaluation were presented. Among the 13 patients with evaluable efficacy, partial response (PR) was observed in 7 patients, ORR was 53.8% (7/13), DCR was 100% (13/13), and 83.5% (11/13) of the patients had shrinkage of tumor target lesions at the first tumor evaluation.

In the previous two-drug combination (IN10018+PLD) treatment study, a total of 52 patients were evaluable for efficacy, of which 24 cases of PR and 1 case of complete response (CR) were observed. The ORR of the best overall efficacy was 48.1%. However, the ORR of the two-drug combination in the treatment of platinum-resistant recurrent ovarian cancer at the first tumor evaluation was 28.8%. Compared with the two-drug combination, three-drug combination (IN10018+PLD+anti-PD-1) treatment significantly improved the efficacy at the first tumor evaluation. More efficacy will need to wait for further maturation of data.

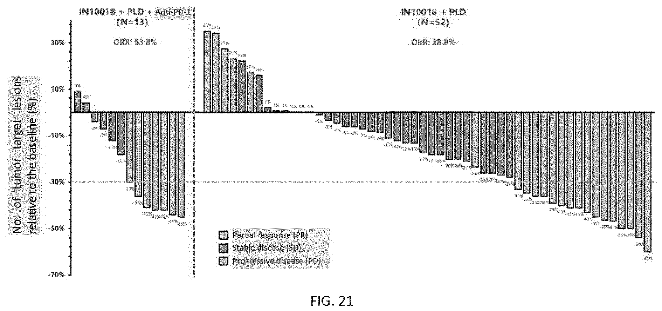

The specific data were shown in Table 13, Table 14 and FIG. 21.

**Table 13: Summary of the anti-tumor efficacy of the two-drug combination and three-drug combination**

| | **IN10018+PLD+anti-PD-1** | **IN10018+PLD** | | | | |
|---|---|---|---|---|---|---|
| **Efficacy evaluation** | **Efficacy at the first tumor evaluation (N=13)** | **Best overall efficacy (N=52)** | **Efficacy at the first tumor evaluation (N=52)** | | | |

| | **N** | **%** | **N** | **%** | **N** | **%** |
|---|---|---|---|---|---|---|
| Complete response (CR) | 0 | 0 | 1 | 1.9% | 0 | 0.0% |
| Partial response (PR) | 7 | 53.8% | 24 | 46.2% | 15 | 28.8% |
| **Objective response rate (ORR)** | 7 | **53.8%** | 25 | 48.1% | 15 | **28.8%** |
| Stable disease (SD) | 6 | 50.0% | 19 | 36.5% | 29 | 55.8% |
| **Disease control rate (DCR)** | 13 | 100.0% | 44 | 84.6% | 44 | 84.6% |
| Progressive disease (PD) | 0 | 0.0% | 8 | 15.4% | 8 | 15.4% |

**Table 14: List of the efficacy of the three-drug combination at the first tumor evaluation**

| **Subject No.** | **Baseline of the tumor target lesion size** | **Tumor target lesion size at the first tumor evaluation** | **Efficacy at the first tumor evaluation** | **Percentage of tumor shrinkage** |
|---|---|---|---|---|
| S0010002 | 32.77 | 22.8 | PR | -30.4% |
| S0010004 | 34.4 | 20 | PR | -41.9% |
| S0030001 | 110.71 | 71.3 | PR | -35.6% |
| S0030002 | 29.49 | 28.1 | SD | -4.7% |
| S0030003 | 25.7 | 14.5 | PR | -43.6% |
| S0030007 | 35.26 | 32.87 | SD | -6.8% |
| S0030008 | 39 | 31.9 | SD | -18.2% |
| S0040004 | 17.7 | 10.3 | PR | -41.8% |
| S0040005 | 79.9 | 82.8 | SD | +3.6% |
| S0070001 | 33.7 | 18.7 | PR | -44.5% |
| S0070002 | 45.04 | 26.64 | PR | -40.9% |
| S0080023 | 77.5 | 74.6 | SD | -3.7% |
| S0080028 | 72.2 | 78.8 | SD | +9.1% |

The optimal change in the total diameter of target lesion relative to the baseline = (the minimum total diameter of target lesion after treatment - baseline of the total diameter of target lesion) / baseline of the total diameter of target lesion x 100%.

### Safety

Safety of the three-drug combination (IN10018+PLD+anti-PD-1) treatment in solid tumors including ovarian cancer was evaluated in the study. A total of 41 patients with solid tumors were enrolled, including 24 patients with platinum-resistant recurrent ovarian cancer.

The safety analysis showed that the safety of the three-drug combination (IN10018+PLD+anti-PD-1) treatment was generally equivalent to that of the two-drug combination (IN10018+PLD) treatment. The safety characteristics were comparable to the historical data of the respective drugs. No significant increase in adverse events was observed due to the combination. Summary of the safety of two-drug and three-drug combinations is shown in Table 15 below.

**Table 15: Summary of the safety of two-drug and three-drug combinations**

| **Number of subjects experiencing AE** | **IN10018 + PLD + PD-1 N=41** | | **IN10018 + PLD N=50** | |
|---|---|---|---|---|
| | N | % | N | % |
| Subjects experiencing at least one AE | 34 | 82.9% | 48 | 96.0% |
| Subjects experiencing IN10018 related | 30 | 73.2% | 47 | 94.0% |
| Subjects experiencing AE of grade ≥3 | 13 | 31.7% | 25 | 50.0% |
| Subjects experiencing IN10018 related AE of grade ≥3 | 7 | 17.1% | 9 | 18.0% |
| Subjects experiencing SAE | 4 | 9.8% | 11 | 22.0% |
| Subjects experiencing IN10018 related SAE | 3 | 7.3% | 5 | 10.0% |
| Subjects experiencing AE of special interest (AESI) | 5 | 12.2% | 5 | 10.0% |
| Death due to AE | 1 | 2.4% | 2 | 4.0% |
| IN10018 discontinuation due to AE | 3 | 7.3% | 2 | 4.0% |
| IN10018 reduction due to AE | 3 | 7.3% | 3 | 6.0% |

All references mentioned in the present disclosure are incorporated by reference in their entirety as if each document was listed separately. It should be understood that after reading the present disclosure, those skilled in the art can make various changes or modifications to the present disclosure, and these equivalents also fall within the scope defined by the claims of this application.

This application claims the priority of Chinese patent application 202111508073.8 filed on December 10, 2021, Chinese patent application 202211160418.X filed on September 22, 2022, and Chinese patent application 202211543036.5 filed on December 2, 2022. The disclosure of the above-mentioned Chinese patent applications is hereby cited in its entirety as part of this application.

## Claims

1. Use of an FAK inhibitor, an anthracycline chemotherapy drug and an immune checkpoint inhibitor in the manufacture of a medicament for treating a tumor in a subject, wherein the FAK inhibitor, the anthracycline chemotherapy drug and the immune checkpoint inhibitor are administered to the subject simultaneously or sequentially.

2. A pharmaceutical combination product of an FAK inhibitor, an anthracycline chemotherapy drug and an immune checkpoint inhibitor, for use in treating a tumor in a subject, wherein the FAK inhibitor, the anthracycline chemotherapy drug and the immune checkpoint inhibitor are administered to the subject simultaneously or sequentially.

3. A method of treating a tumor, comprising administering a therapeutically effective amount of an FAK inhibitor, an anthracycline chemotherapy drug and an immune checkpoint inhibitor simultaneously or sequentially to a subject in need.

4. The use, the pharmaceutical combination product, or the method according to any one of claims 1 to 3, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, wherein the IN 10018 has a structure of:

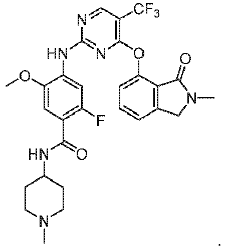

5. The use, the pharmaceutical combination product, or the method according to any one of claims 1 to 4, wherein the anthracycline chemotherapy drug is Adriamycin, epirubicin, or daunorubicin, alternatively, the anthracycline chemotherapy drug is Adriamycin.

6. The use, the pharmaceutical combination product, or the method according to any one of claims 1 to 5, wherein the immune checkpoint inhibitor is an anti-PD-1/PD-L1 antibody, a PD-1/PD-L1 small molecule inhibitor, or a TIGIT inhibitor.

7. The use, the pharmaceutical combination product, or the method according to any one of claims 1 to 6, wherein the FAK inhibitor is IN10018 or a pharmaceutically acceptable salt thereof, the anthracycline chemotherapy drug is Adriamycin, and the immune checkpoint inhibitor is an anti-PD-1/PD-L1 antibody or a PD-1/PD-L1 small molecule inhibitor, alternatively, the immune checkpoint inhibitor is toripalimab.

8. The use, the pharmaceutical combination product, or the method according to any one of claims 1 to 7, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colon cancer, rectal cancer, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colon cancer, esophageal cancer, hepatocellular carcinoma, ovarian cancer, fibrosarcoma, gastric cancer, non-small cell lung cancer, or cholangiocarcinoma; yet further, the tumor is ovarian cancer or colon cancer.

9. A kit or a pharmaceutically acceptable composition, comprising:
(a) an FAK inhibitor;
(b) an anthracycline chemotherapy drug; and
(c) an immune checkpoint inhibitor.

10. The kit or composition according to claim 9, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, wherein the IN10018 has a structure of:

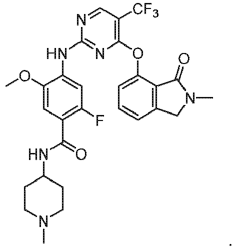

11. The kit or composition according to claim 9 or 10, wherein the anthracycline chemotherapy drug is Adriamycin, epirubicin, or daunorubicin, alternatively, the anthracycline chemotherapy drug is Adriamycin.

12. The kit or composition according to any one of claims 9 to 11, wherein the immune checkpoint inhibitor is an anti-PD-1/PD-L1 antibody, a PD-1/PD-L1 small molecule inhibitor, or a TIGIT inhibitor.

13. The kit or composition according to any one of claims 9 to 12, wherein the FAK inhibitor is IN10018 or a pharmaceutically acceptable salt thereof, the anthracycline chemotherapy drug is Adriamycin, and the immune checkpoint inhibitor is an anti-PD-1/PD-L1 antibody or a PD-1/PD-L1 small molecule inhibitor, alternatively, the immune checkpoint inhibitor is toripalimab.

14. The kit or composition according to any one of claims 9 to 13, for use as a medicament.

15. An FAK inhibitor, for use in enhancing immunogenic cell death induced by an anthracycline chemotherapy drug in the treatment of a tumor.

16. The use, the FAK inhibitor, or the method according to claim 15, wherein the anthracycline chemotherapy drug is Adriamycin, epirubicin, or daunorubicin, alternatively, the anthracycline chemotherapy drug is Adriamycin.

17. The use, the FAK inhibitor, or the method according to claim 15 or 16, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, wherein the IN10018 has a structure of:

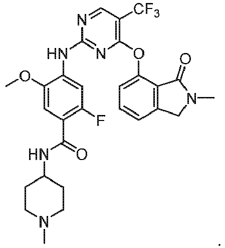

18. Use of an FAK inhibitor, Adriamycin and an immune checkpoint inhibitor in the manufacture of a medicament for treating a tumor in a subject, wherein the FAK inhibitor, the Adriamycin and the immune checkpoint inhibitor are administered to the subject simultaneously or sequentially.

19. A pharmaceutical combination product of an FAK inhibitor, Adriamycin and an immune checkpoint inhibitor, for use in treating a tumor in a subject, wherein the FAK inhibitor, the Adriamycin and the immune checkpoint inhibitor are administered to the subject simultaneously or sequentially.

20. A method of treating a tumor, comprising administering a therapeutically effective amount of an FAK inhibitor, Adriamycin and an immune checkpoint inhibitor simultaneously or sequentially to a subject in need.

21. The use, the pharmaceutical combination product, or the method according to any one of claims 18 to 20, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018 or a pharmaceutically acceptable salt thereof, alternatively, the FAK inhibitor is IN10018 tartrate, wherein the IN 10018 has a structure of:

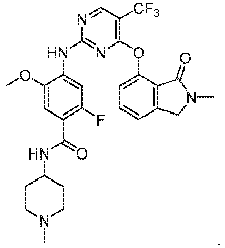

22. The use, the pharmaceutical combination product, or the method according to any one of claims 18 to 21, wherein the immune checkpoint inhibitor is an anti-PD-1/PD-L1 antibody, a PD-1/PD-L1 small molecule inhibitor, or a TIGIT inhibitor.

23. The use, the pharmaceutical combination product, or the method according to any one of claims 18 to 22, wherein the FAK inhibitor is IN10018 or a pharmaceutically acceptable salt thereof, and the immune checkpoint inhibitor is an anti-PD-1/PD-L1 antibody or a PD-1/PD-L1 small molecule inhibitor, alternatively, the immune checkpoint inhibitor is toripalimab.

24. The use, the pharmaceutical combination product, or the method according to any one of claims 18 to 23, wherein the tumor is Hodgkin lymphoma, non-Hodgkin lymphoma, non-small cell lung cancer, small cell lung cancer, hepatocellular carcinoma, cholangiocarcinoma, myelodysplastic syndrome, acute lymphoblastic leukemia, acute myeloid leukemia, chronic myeloid leukemia, thyroid cancer, glioma, colon cancer, rectal cancer, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, gastroesophageal junction cancer, thymic carcinoma, pancreatic cancer, endometrial cancer, cervical cancer, melanoma, uveal melanoma, skin cancer, germ cell tumor, nasopharyngeal carcinoma, oropharyngeal cancer, or laryngeal cancer; further, the tumor is acute myeloid leukemia, melanoma, thyroid cancer, colon cancer, esophageal cancer, hepatocellular carcinoma, ovarian cancer, fibrosarcoma, gastric cancer, non-small cell lung cancer, or cholangiocarcinoma; yet further, the tumor is ovarian cancer or colon cancer.
